# EUROPÄISCHE PATENTSCHRIFT

(11) **EP 0 588 137 B1**
(45) Veröffentlichungstag und Bekanntmachung des Hinweises auf die Patenterteilung: **03.02.1999**
(21) Anmeldenummer: 93113815.0
(22) Anmeldetag: 30.08.1993
(51) Int. Cl.: C07D 309/38, C07D 311/74, C07D 311/94, C07D 405/04, C07D 405/06, C07D 407/04, C07D 407/06, C07D 409/04, C07D 413/06, C07D 493/04, C07D 495/04, A01N 43/16

(54) **3-Aryl-pyron-Derivate**
3-Aryl-2-pyranone derivatives
Dérivés de la 3-aryl-pyrane-2-one

(30) Priorität: 10.09.1992 DE 4230267; 17.03.1993 DE 4308451
(43) Veröffentlichungstag der Anmeldung: 23.03.1994
(73) Patentinhaber: BAYER AG, 51368 Leverkusen (DE)
(72) Erfinder: Fischer, Reiner, Dr., D-40789 Monheim (DE); Krebs, Andreas, Dr., D-51519 Odenthal (DE); Lieb, Folker, Dr., D-51375 Leverkusen (DE); Ruther, Michael, Dr., D-40789 Monheim (DE); Stetter, Jörg, Dr., D-42115 Wuppertal (DE); Erdelen, Christoph, Dr., D-42799 Leichlingen (DE); Wachendorff-Neumann, Ulrike, D-40789 Monheim (DE); Lürssen, Klaus, Dr., D-51469 Bergisch Gladbach (DE); Santel, Hans-Joachim, Dr., D-51371 Leverkusen (DE); Schmidt, Robert R., Dr., D-51467 Bergisch Gladbach (DE)

(56) Entgegenhaltungen:
- EP-A- 0 483 582
- DE-A- 1 593 373
- CHEMICAL ABSTRACTS, vol. 57, no. 10, 12. November 1962, Columbus, Ohio, US; abstract no. 12418b, C. GOETSCHEL ET AL.
- CHEMICAL ABSTRACTS, vol. 109, no. 19, 7. November 1988, Columbus, Ohio, US; abstract no. 170174w, G. DANNHARDT ET AL.
- CHEMICAL ABSTRACTS, vol. 86, no. 23, 6. Juni 1977, Columbus, Ohio, US; abstract no. 167911k, M. KLAAR ET AL.

## Beschreibung

Die Erfindung betrifft neue 3-Aryl-pyron-Derivate, Verfahren zu ihrer Herstellung und ihre Verwendung als Schädlingsbekämpfungsmittel.

Bestimmte im Phenylring unsubstituierte Phenyl-pyron-Derivate sind bereits bekannt geworden (vgl. A.M. Chirazi, T. Kappe und E. Ziegler, Arch. Pharm. 309, 558 (1976) und K.-H. Boltze und K. Heidenbluth, Chem. Ber. 91, 2849), wobei für diese Verbindungen eine mögliche Verwendbarkeit als Schädlingsbekämpfungsmittel nicht angegeben wird.

In Chemical Abstracts, Band 109, 1988, Nr. 170174w werden Indolizin-5-on-Derivate und deren Verwendung als Bakterizide offenbart.

Es wurden nun die neuen substituierten 3-Aryl-pyron-Derivate der allgemeinen Formel (I) 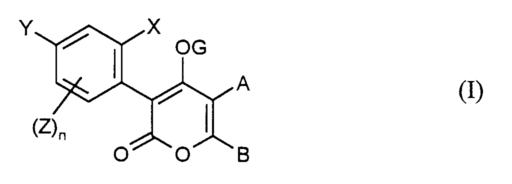 gefunden, in welcher
- A: für Wasserstoff, Halogen, gegebenenfalls substituierte Reste aus der Reihe Alkyl, Cycloalkyl, Alkenyl, Alkinyl, Arylalkyl, Aryl, Hetarylalkyl und Hetaryl oder für die Gruppen -COR¹, -CO₂R¹, -CN, -CONR¹R², -SO₂R¹ und -P(O)(OR¹)(OR²) steht, worin
- R¹ und R²: unabhängig voneinander für Wasserstoff oder gegebenenfalls substituierte Reste aus der Reihe Alkyl, Alkenyl, Arylalkyl, Aryl, Hetarylalkyl und Hetaryl stehen oder
- R¹ und R²: gemeinsam für eine gegebenenfalls substituierte Alkylen-Gruppe stehen, welche durch ein oder mehrere Heteroatome unterbrochen sein kann;
- B: für Wasserstoff oder gegebenenfalls substituierte Reste aus der Reihe Alkyl, Cycloalkyl, Alkenyl, Alkinyl, Arylalkyl, Aryl, Hetarylalkyl und Hetaryl stehen;
- A und B: gemeinsam eine gegebenenfalls substituierte Alkylen- oder Alkenylen-Gruppe bilden, welche durch ein oder mehrere Heteroatome oder Heterogruppen unterbrochen sein kann bzw. diese enthalten kann;
- X: für Halogen, Alkyl oder Alkoxy steht;
- Y: für Wasserstoff, Halogen, Alkyl, Halogenalkyl oder Alkoxy steht;
- Z: für Wasserstoff, Halogen, Alkyl oder Alkoxy steht,
- n: für eine ganze Zahl 1, 2 oder 3 steht; und
- G: für Wasserstoff, ein Metallionenäquivalent, ein Ammonium oder für eine Gruppe -COR³, 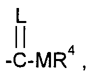 -SO₂-R⁵, 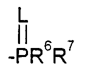 und -CONR⁸R⁹ steht, worin
- R³: für gegebenenfalls substituierte Reste aus der Reihe Alkyl, Alkenyl, Alkoxyalkyl, Polyalkoxyalkyl, Alkylthioalkyl gegebenenfalls durch ein oder mehrere Heteroatome unterbrochenes Cycloalkyl, Arylalkyl, Aryl, Hetarylalkyl, Hetaryl, Aryloxyalkyl und Hetaryloxyalkyl steht;
- R⁴: für gegebenenfalls substituierte Reste aus der Reihe Alkyl, Alkenyl, Alkoxyalkyl, Polyyalkoxyalkyl, Aryl und Arylalkyl steht;
- R⁵, R⁶ und R⁷: unabhängig voneinander für gegebenenfalls substituierte Reste aus der Reihe Alkyl, Alkoxy, Alkylthio, Alkylamino, Dialkylamino, Alkenylthio, Alkinylthio, Cycloalkylthio, Aryl, Aryloxy und Arylthio stehen;
- R⁸ und R⁹: unabhängig voneinander für Wasserstoff oder für gegebenenfalls substituierte Reste der Reihe Alkyl, Alkenyl, Cycloalkyl, Alkoxyalkyl, Aryl oder Arylalkyl stehen, oder gemeinsam eine gegebenenfalls substituierte Alkylen-Gruppe bilden, welche durch ein oder mehrere Heteroatome oder Heterogruppen unterbrochen sein kann;
- L: für Sauerstoff oder Schwefel steht; und
- M: für Sauerstoff oder Schwefel steht,
ausgenommen die Verbindung der Formel

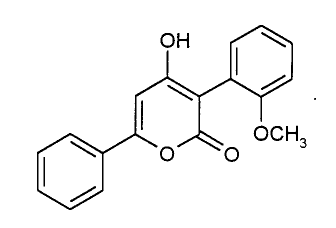

Die Verbindungen der allgemeinen Formel (I) können in Abhängigkeit der Substituenten als geometrische und/oder optische Isomere oder Isomerengemische unterschiedlicher Zusammensetzung vorliegen. Sowohl die reinen Isomeren als auch die Isomerengemische, deren Herstellung und Verwendung sowie diese enthaltende Mittel sind Bestandteile der beanspruchten Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Weiterhin wurde gefunden, daß die neuen Verbindungen der allgemeinen Formel (I) eine sehr gute Wirksamkeit als Schädlingsbekämpfungsmittel, vorzugsweise als Arthropodizide, Nematizide und Herbizide sowie als Ekto- und Endoparasitizide aufweisen.

Weiterhin wurde gefunden, daß man die neuen 3-Aryl-pyron-Derivate der allgemeinen Formel (I) erhält, wenn man
a) zur Herstellung der Verbindungen, in welchen G für Wasserstoff steht,
   Carbonylverbindungen der allgemeinen Formel (II) 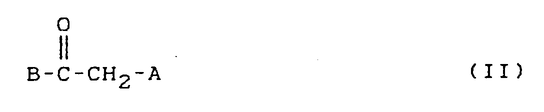 in welcher
   A und B die oben angegebene Bedeutung haben,
   mit Ketensäurehalogeniden der allgemeinen Formel (III) 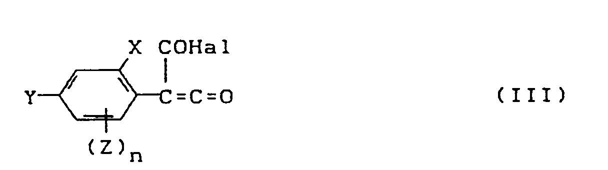 in welcher
   X, Y, Z und n die oben angegebenen Bedeutungen haben und
   - Hal: für Halogen (vorzugsweise für Chlor oder Brom) steht
   in Gegenwart eines Verdünnungsmittels und gegebenenfalls in Gegenwart eines Säureakzeptors umsetzt; und
b) zur Herstellung der Verbindungen der allgemeinen Formel (I), in welcher G für -COR³ steht,
   Verbindungen der allgemeinen Formel (I), in welchen G für Wasserstoff steht (erhältlich nach Variante a))
   α) mit Säurehalogeniden der allgemeinen Formel (IV) 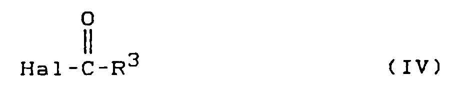 in welcher
      R³ die oben angegebene Bedeutung hat und
      Hal für Halogen (vorzugsweise für Chlor oder Brom) steht, oder
   β) mit Carbonsäureanhydriden der allgemeinen Formel (V) 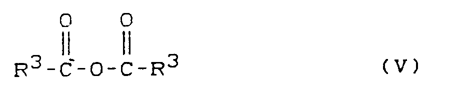 in welcher
      R³ die oben angegebene Bedeutung hat,
   gegebenenfalls in Gegenwart eines Verdünnungsmittels und gegebenenfalls in Gegenwart eines Säurebindemittels umsetzt; und
c) zur Herstellung der Verbindungen der allgemeinen Formel (I), in welchen G für -C(L)-MR⁴ steht, worin L Sauerstoff bedeutet,
   Verbindungen der allgemeinen Formel (I), in welchen G für Wasserstoff steht (erhältlich nach Variante a)),
   mit Verbindungen der allgemeinen Formel (VI) 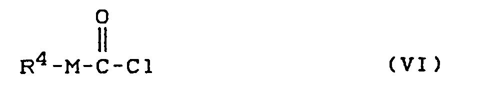 in welcher
   R⁴ und M die oben angegebenen Bedeutungen haben,
   gegebenenfalls in Gegenwart eines Verdünnungsmittels und gegebenenfalls in Gegenwart eines Säurebindemittels umsetzt; und
d) zur Herstellung der Verbindungen der allgemeinen Formel (I), in welchen G für -C(L)-MR⁴ steht, worin L Schwefel bedeutet,
   Verbindungen der allgemeinen Formel (I), in welchen G für Wasserstoff steht (erhältlich nach Variante a)),
   α) mit Verbindungen der allgemeinen Formel (VII) 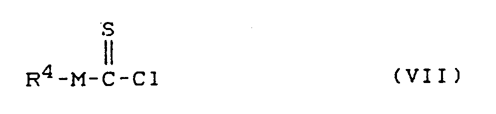 in welcher
      R⁴ und M die oben angegebenen Bedeutungen haben,
      gegebenenfalls in Gegenwart eines Verdünnungsmittels und gegebenenfalls in Gegenwart eines Säurebindemittels umsetzt, oder
   β) mit Schefelkohlenstoff (CS₂) und anschließend mit Alkylhalogeniden der allgemeinen Formel (VIII)

      R⁴-Hal¹ (VIII)

      in welcher
      R⁴ die oben angegebene Bedeutung hat und
      - Hal¹: für Halogen (vorzugsweise für Chlor, Brom und Iod) steht,
      gegebenenfalls in Gegenwart eines Verdünnungsmittels und gegebenenfalls in Gegenwart eines Säurebindemittels umsetzt; und
e) zur Herstellung von Verbindungen der allgemeinen Formel (I), in welcher G für -SO₂R⁵ steht,
   Verbindungen der allgemeinen Formel (I), in welchen G für Wasserstoff steht (erhältlich nach Variante a)),
   mit Sulfonsäurechloriden der allgemeinen Formel (IX)

   R⁵-SO₂-Cl (IX)

   in welcher
   R⁵ die oben angegebene Bedeutung hat,
   gegebenenfalls in Gegenwart eines Verdünnungsmittels und gegebenenfalls in Gegenwart eines Säurebindemittels umsetzt; und
f) zur Herstellung von Verbindungen der allgemeinen Formel (I), in welchen G für -P(L)R⁶R⁷ steht,
   Verbindungen der allgemeinen Formel (I), in welchen G für Wasserstoff steht (erhältlich nach Variante a)),
   mit Verbindungen der allgemeinen Formel (X) 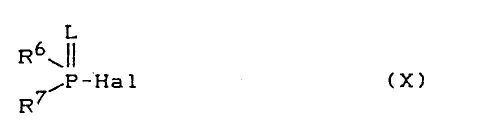 in welcher
   R⁶, R⁷ und L die oben angegebenen Bedeutungen haben und
   Hal für Halogen (vorzugsweise Chlor oder Brom) steht,
   gegebenenfalls in Gegenwart eines Verdünnungsmittels und gegebenenfalls in Gegenwart eines Säurebindemittels umsetzt; und
g) zur Herstellung von Verbindungen der allgemeinen Formel (I), in welchen G für ein Metallionenäquivalent oder für ein Ammoniumion steht,
   Verbindungen der allgemeinen Formel (I), in welchen G für Wasserstoff steht (erhältlich nach Variante a)),
   mit
   Metallhydroxiden oder Aminen (vorzugsweise Mono-, Di- oder Trialkylaminen)
   umsetzt; und
h) zur Herstellung von Verbindungen der allgemeinen Formel (I), in welchen G für -C(L)NR⁸R⁹ steht,
   Verbindungen der allgemeinen Formel (I), in welchen G für Wasserstoff steht (erhältlich nach Variante a)),
   α) mit Verbindungen der allgemeinen Formel (XI)

      R⁸-N=C=L (XI)

      in welcher
      R⁸ und L die oben angegebenen Bedeutungen haben,
      gegebenenfalls in Gegenwart eines Verdünnungsmittels und gegebenenfalls in Gegenwart eines Katalysators umsetzt; oder
   β) mit Verbindungen der allgemeinen Formel (XII) 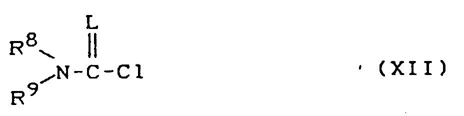 in welcher
      R⁸, R⁹ und L die oben angegebenen Bedeutungen haben,
      gegebenenfalls in Gegenwart eines Verdünnungsmittels und gegebenenfalls in Gegenwart eines Säurebindemittels umsetzt.

Alkyl als solches oder als Bestandteil einer anderen Gruppe (z.B, Alkoxy, Alkylthio und Halogenalkyl) bedeutet in den allgemeinen Formeln geradkettiges oder verzweigtes Alkyl mit vorteilhaft 1 bis 20, besonders vorteilhaft 1 bis 18 und ganz besonders vorteilhaft 1 bis 16 Kohlenstoffatomen. Alkyl enthält vorzugsweise 1 bis 8, besonders bevorzugt 1 bis 6 und ganz besonders bevorzugt 1 bis 4 Kohlenstoffatome, wobei Methyl, Ethyl, n- und i-Propyl sowie n-, i-, s- und t-Butyl speziell genannt seien.

Cycloalkyl als solches oder als Bestandteil einer anderen Gruppe (z.B. Cycloalkylthio) enthält in den allgemeinen Formeln vorzugsweise 3 bis 10, besonders bevorzugt 3 bis 7 und ganz besonders bevorzugt 3 bis 6 Kohlenstoffatome, wobei Cyclopropyl, Cyclobutyl, Cyclopentyl und Cyclohexyl speziell genannt seien. Im Falle, daß Cycloalkyl durch ein oder mehrere Heteroatome oder Heterogruppen unterbrochen ist, handelt es sich um gleiche oder verschiedene, vorzugsweise 1 oder 2 Heteroatome bzw. Heterogruppen. Als Heteroatome stehen vorzugsweise Sauerstoff oder Schwefel und als Heterogruppen vorzugsweise NH oder NC₁-C₄-Alkyl.

Alkenyl und Alkinyl als solche oder als Bestandteil einer anderen Gruppe (wie Alkenylthio und Alkinylthio) bedeuten in den allgemeinen Formeln geradkettiges oder verzweigtes Alkenyl und Alkinyl mit vorzugsweise 1 Doppel- bzw. Dreifachbindung und vorzugsweise 2 bis 8, insbesondere 3 bis 6 und ganz besonders bevorzugt 3 oder 4 Kohlenstoffatomen, wobei die Allyl- und Propargylgruppen speziell genannt seien.

Aryl als solches oder als Bestandteil einer anderen Gruppe (wie Aryloxy oder Arylthio) bedeutet in den allgemeinen Formeln vorzugsweise Naphthyl und Phenyl, besonders bevorzugt Phenyl.

Aralkyl bedeutet in den allgemeinen Formeln vorzugsweise Naphthylalkyl oder Phenylalkyl, besonders bevorzugt Phenylalkyl. Der Alkylteil ist geradkettig oder verzweigt und enthält vorzugsweise 1 bis 6, besonders bevorzugt 1 bis 4 und ganz besonders bevorzugt 1 oder 2 Kohlenstoffatomen. Speziell seien Benzyl und Phenylethyl genannt.

Hetaryl als solches oder als Bestandteil einer anderen Gruppe (wie Hetarylalkyl oder Hetaryloxyalkyl) bedeutet in den allgemeinen Formeln heteroaromatische 5- bis 7-gliedrige Ringe mit vorzugsweise 1 bis 3, insbesondere 1 oder 2 gleichen oder verschiedenen Heteroatomen. Als Heteroatome stehen vorzugsweise Sauerstoff, Schwefel oder Stickstoff. Beispielhaft und vorzugsweise seien Pyrryl, Furyl, Thienyl, Thiazolyl, Pyridyl, Pyrazolyl und Pyrimidinyl genannt.

In Hetarylalkyl und Hetaryloxyalkyl der allgemeinen Formeln hat Hetaryl die vorstehend angegebene Bedeutung. Die Alkylbestandteile sind geradkettig oder verzweigt und enthalten vorzugsweise 1 bis 6, besonders bevorzugt 1 bis 4 und ganz besonders bevorzugt 1 oder 2 Kohlenstoffatome, wobei Hetarylmethyl und Hetaryloxymethyl speziell genannt seien.

In Alkylthio, Alkoxy, Halogenalkyl, Halogenalkoxy, Alkoxyalkyl, Alkylthioalkyl, Polyalkoxyalkyl, Aryloxyalkyl, Hetaryloxyalkyl, Alkylamino und Dialkylamino haben die Alkylteile die oben bei Alkyl angegebenen Bedeutungen.

Halogenalkyl und Halogenalkoxy enthalten vorzugsweise 1 bis 8, insbesondere 1 bis 5 und ganz besonders bevorzugt 1 bis 5 gleiche oder verschiedene Halogenatome. Als Halogenatome stehen vorzugsweise Fluor, Chlor, Brom und/oder Iod, insbesondere Fluor, Chlor und/oder Brom und ganz besonders bevorzugt Fluor und/oder Chlor. Beispielhaft seien Trifluormethyl, Chlor-difluormethyl, Brommethyl, 2,2,2-Trifluorethyl und Pentafluorethyl genannt.

In Aryloxyalkyl und Hetaryloxyalkyl der allgemeinen Formeln haben die Aryl- bzw. Hetarylteile die oben für diese Reste angegebenen Bedeutungen.

In Alkenylthio, Alkinylthio und Cycloalkylthio der allgemeinen Formeln haben Alkenyl, Alkinyl und Cycloalkyl die oben bei den jeweiligen Resten angegebenen Bedeutungen.

In Aryloxy und Arylthio der allgemeinen Formeln haben die Arylteile die oben für Aryl angegebenen Bedeutungen.

In den Polyalkoxyresten der allgemeinen Formeln sind vorzugsweise 2 bis 4, insbesondere 2 bis 3 und ganz besonders bevorzugt 2 Alkoxyreste enthalten wobei die Alkylteile die oben angegebene Bedeutung haben.

Wenn zwei Substituenten der allgemeinen Formeln (z.B. A und B, R¹ und R² sowie R⁸ und R⁹) gemeinsam eine Alkylen- oder Alkenylen-Gruppe bilden, ist diese Gruppe geradkettig oder verzweigt und enthält vorzugsweise 2 bis 7, insbesondere 2 bis 6 und ganz besonders bevorzugt 2 bis 5 Kohlenstoffatome. Die Alkenylen-Gruppen enthalten eine oder mehrere, vorzugsweise 1 oder 2, insbesondere 1 Doppelbindung. Die Alkylen- oder AlkenylenGruppen können durch ein oder mehrere, gleiche oder verschiedene Heteroatome oder Heterogruppen, wie Sauerstoff, Schwefel oder Stickstoff und -OCO- unterbrochen sein bzw. solche Gruppen enthalten. Beispielhaft seien -(CH₂)₄-, -(CH₂)₅-, -(CH₂)₂-O-(CH₂)₂- und -(CH₂)₂-S-(CH₂)₂- oder -O-CO-CH₂- genannt.

Halogen bedeutet in den allgemeinen Formeln, wenn nicht anders definiert, Fluor, Chlor, Brom und/oder Iod, vorzugsweise Fluor, Chlor und/oder Brom und ganz besonders bevorzugt Fluor und/oder Chlor.

Ein Metallionenäquivalent bedeutet in den allgemeinen Formeln ein Äquivalent eines Metallkations, vorzugsweise des Kations eines Erdalkali- oder Alkalimetalls, insbesondere eines Kalzium-, Magnesium-, Natrium- oder Kaliumkations, ganz besonders bevorzugt eines Kalzium-, Natrium- oder Kaliumkations.

Ein Ammoniumion bedeutet in den allgemeinen Formeln vorzugsweise ein Monoalkyl-, Dialkyl- oder Trialkylammoniumion, wobei die Alkylreste vorzugsweise 1 bis 6, insbesondere 1 bis 4 und ganz besonders bevorzugt 1 oder 2 Kohlenstoffatome enthalten. Die Alkylreste können ein oder mehrfach, vorzugsweise einfach, substituiert vorliegen, wobei als Substituenten vorzugsweise Hydroxy oder Halogen genannt seien.

n steht in den allgemeinen Formeln vorzugsweise für 1 oder 2, besonders bevorzugt für 1, wobei der Substituent Z sich vorzugsweise in der 6-Stellung des Phenylringes befindet.

Die in den allgemeinen Formeln aufgeführten gegebenenfalls substituierten Reste können einen oder mehrere, vorzugsweise 1 bis 3, insbesondere 1 oder 2 gleiche oder verschiedene Substituenten tragen. Als Substitenten seien beispielhaft und vorzugsweise aufgeführt: Alkyl mit vorzugsweise 1 bis 8, insbesondere 1 bis 6 und ganz besonders bevorzugt 1 bis 4 Kohlenstoffatomen, wie Methyl, Ethyl, n- und i-Propyl und n-, i- und t-Butyl; Alkoxy mit vorzugsweise 1 bis 8, insbesondere 1 bis 6 und ganz besonders bevorzugt 1 bis 4 Kohlenstoffatomen, wie Methoxy, Ethoxy, n- und i-Propyloxy und n-, i- und t-Butyloxy; Alkylthio mit vorzugsweise 1 bis 8, insbesondere 1 bis 6 und ganz besonders bevorzugt 1 bis 4 Kohlenstoffatomen, wie Methylthio, Ethylthio, n- und i-Propylthio und n-, i- und t-Butylthio; Halogenalkyl und Halogenalkoxy mit vorzugsweise 1 bis 8, insbesondere 1 bis 6 und ganz besonders bevorzugt 1 bis 4 Kohlenstoffatomen und vorzugsweise 1 bis 7, insbesondere 1 bis 5 und ganz besonders bevorzugt 1 bis 3 Kohlenstoffatomen, wobei die Halogenatome gleich oder verschieden sind und als Halogenatome, vorzugsweise Fluor, Chlor oder Brom, insbesondere Fluor stehen, wie Trifluormethyl oder Trifluormethoxy; Hydroxy; Amino; Halogen, vorzugsweise Fluor, Chlor, Brom und Iod, insbesondere Fluor, Chlor und Brom; Cyano, Nitro, gegebenenfalls durch die genannten Reste substituiertes Phenyl; Alkylcarbonyloxy mit vorzugsweise 1 bis 6, insbesondere 1 bis 4 und ganz besonders bevorzugt 1 oder 2 Kohlenstoffatomen in der Alkylgruppe oder ein heteroaliphatischer oder heteroaromatischer Rest, wie Pyridyl, Furyl oder Tetrahydrofuryl.

A steht in den allgemeinen Formeln bevorzugt für Wasserstoff; Halogen insbesondere Chlor oder Fluor; gegebenenfalls durch Halogen substituierte Reste aus der Reihe C₁-C₁₀-Alkyl, C₃-C₁₀-Alkenyl, C₃-C₁₀-Alkinyl und C₃-C₁₀-Cycloalkyl; für gegebenenfalls durch Halogen, Nitro, C₁-C₈-Alkyl, C₁-C₈-Alkoxy, C₁-C₈-Alkylthio, C₁-C₈-Halogenalkyl, C₁-C₈-Halogenalkoxy und/oder CN substituierte Reste aus der Reihe Phenyl, Naphthyl, Hetaryl, Phenyl-C₁-C₆-alkyl und Hetaryl-C₁-C₆-alkyl oder für die Gruppen -COR¹, -CO₂R¹, -CN, -CONR¹R², -SO₂R¹ und -P(O)(OR¹)(OR²), wobei
R¹ und R² unabhängig voneinander für Wasserstoff, für gegebenenfalls durch Halogen substituierte Reste aus der Reihe C₁-C₁₀-Alkyl und C₃-C₁₀-Alkenyl; für gegebenenfalls durch Halogen, Nitro, C₁-C₈-Alkyl, C₁-C₈-Alkoxy, C₁-C₈-Alkylthio, C₁-C₈-Halogenalkyl, C₁-C₈-Halogenalkoxy und/oder CN substituierte Reste aus der Reihe Phenyl, Naphthyl, Hetaryl, Phenyl-C₁-C₆-alkyl und Hetaryl-C₁-C₆-alkyl stehen; oder R¹ und R² gemeinsam für eine gegebenenfalls durch Stickstoff, Sauerstoff oder Schwefel unterbrochene C₂-C₇-Alkylen-Gruppe stehen.

A steht in den allgemeinen Formeln besonders bevorzugt für Wasserstoff; Halogen; gegebenenfalls durch Halogen substituierte Reste aus der Reihe C₁-C₈-Alkyl, C₃-C₈-Alkenyl, C₃-C₈-Alkinyl und C₃-C₈-Cycloalkyl; für gegebenenfalls durch Halogen, Nitro, C₁-C₄-Alkyl, C₁-C₄-Alkoxy, C₁-C₄-Alkylthio, C₁-C₄-Halogenalkyl, C₁-C₄-Halogenalkoxy und/oder CN substituierte Reste aus der Reihe Phenyl, Naphthyl, Hetaryl, Phenyl-C₁-C₄-alkyl und Hetaryl-C₁-C₄-alkyl oder für die Gruppen -COR¹, -CO₂R¹, -CN, -CONR¹R², -SO₂R¹ und -P(O)(OR¹)(OR²), wobei
R¹ und R² unabhängig voneinander für Wasserstoff, für gegebenenfalls durch Halogen substituierte Reste aus der Reihe C₁-C₈-Alkyl und C₃-C₈-Alkenyl; für gegebenenfalls durch Halogen, Nitro, C₁-C₄-Alkyl, C₁-C₄-Alkoxy, C₁-C₄-Alkylthio, C₁-C₄-Halogenalkyl, C₁-C₄-Halogenalkoxy und/oder CN substituierte Reste aus der Reihe Phenyl, Hetaryl, Phenyl-C₁-C₄-alkyl und Hetaryl-C₁-C₄-alkyl stehen; oder R¹ und R² gemeinsam für eine gegebenenfalls durch Stickstoff, Sauerstoff oder Schwefel unterbrochene C₂-C₆-Alkylen-Gruppe stehen.

A steht in den allgemeinen Formeln ganz besonders bevorzugt für Wasserstoff; Halogen; gegebenenfalls durch Halogen substituierte Reste aus der Reihe C₁-C₆-Alkyl, C₃-C₆-Alkenyl, C₃-C₆-Alkinyl und C₃-C₆-Cycloalkyl; für gegebenenfalls durch Halogen, Nitro, C₁-C₄-Alkyl, C₁-C₄-Alkoxy, C₁-C₄-Alkylthio, C₁-C₄-Halogenalkyl, C₁-C₄-Halogenalkoxy und/oder CN substituierte Reste aus der Reihe Phenyl, Phenyl-C₁-C₂-alkyl, Thienyl, Furyl, Thiazolyl, Pyridyl und Pyrazolyl oder für die Gruppen -COR¹, -CO₂R¹, -CN, -CONR¹R², -SO₂R¹ und
-P(O)(OR¹)(OR²), wobei
R¹ und R² unabhängig voneinander für Wasserstoff, für gegebenenfalls durch Halogen substituierte Reste aus der Reihe C₁-C₆-Alkyl und C₃-C₆-Alkenyl; für gegebenenfalls durch Halogen, Nitro, C₁-C₄-Alkyl, C₁-C₄-Alkoxy, C₁-C₄-Alkylthio, C₁-C₄-Halogenalkyl, C₁-C₄-Halogenalkoxy und/oder CN substituiertes Phenyl stehen.

In einer besonders hervorgehobenen Ausführungsform der vorliegenden Erfindung steht A für Wasserstoff, Chlor, Fluor, gegebenenfalls durch Halogen substituierte Reste aus der Reihe C₁-C₄-Alkyl, Phenyl und Benzyl oder A und B bilden gemeinsam eine gegebenenfalls durch Sauerstoff oder Schwefel unterbrochene C₃-C₅-Alkenylgruppe (vorzugsweise -CH₂-CH(CH₃)-(CH₂)₂-, -(CH₂)₃-, -(CH₂)₄-, -(CH₂)₅-, -CH₂O(CH₂)₂- oder CH₂-S(CH₂)₂).

B steht in den allgemeinen Formeln bevorzugt für Wasserstoff; für gegebenenfalls durch Halogen, CN, C₁₋₆-Alkoxy, C₁₋₆-Alkylthio, C₁-C₆-Alkylcarbonyloxy oder Phenyl substituierte Reste aus der Reihe C₁-C₁₀-Alkyl, C₃-C₁₀-Alkenyl, C₃-C₁₀-Alkinyl und C₃-C₁₀-Cycloalkyl oder für gegebenenfalls durch Halogen, CN, Nitro, C₁-C₈-Alkyl, C₁-C₈-Alkoxy, C₁-C₈-Alkylthio, C₁-C₈-Halogenalkyl und/oder C₁-C₈-Halogenalkoxy substituierte Reste aus der Reihe Phenyl, Naphthyl, Hetaryl, Phenyl-C₁-C₆-alkyl und Hetaryl-C₁-C₆-alkyl; und
A und B in den allgemeinen Formeln bilden gemeinsam bevorzugt eine gegebenenfalls durch C₁-C₈-Alkyl, C₁₋₆-Halogenalkyl, C₁₋₆-Alkoxy, C₁₋₆-Alkylthio und/oder Halogen substituierte C₂-C₇-Alkylen- oder C₂-C₇-Alkenylen-Gruppe, welche durch Stickstoff, Schwefel, Sauerstoff oder -O-CO- unterbrochen sein kann bzw. diese enthalten kann.

B steht in den allgemeinen Formeln besonders bevorzugt für Wasserstoff; für gegebenenfalls durch Halogen, CN, C₁₋₄-Alkyl, C₁₋₄-Alkylthio, C₁-C₄-Alkylcarbonyloxy oder Phenyl substituierte Reste aus der Reihe C₁-C₈-Alkyl, C₃-C₈-Alkenyl, C₃-C₈-Alkinyl und C₃-C₈-Cycloalkyl oder für gegebenenfalls durch Halogen, CN, Nitro, C₁-C₆-Alkyl, C₁-C₆-Alkoxy, C₁-C₆-Alkylthio, C₁-C₆-Halogenalkyl und/oder C₁-C₆-Halogenalkoxy substituierte Reste aus der Reihe Phenyl, Naphthyl, Hetaryl, Phenyl-C₁-C₄-alkyl und Hetaryl-C₁-C₄-alkyl; und
A und B in den allgemeinen Formeln bilden gemeinsam bevorzugt eine gegebenenfalls durch C₁-C₄-Alkyl, 1-5 Halogen-C₁₋₄-alkyl, C₁₋₄-Alkoxy und/oder Halogen substituierte C₂-C₆-Alkylen- oder C₂-C₆-Alkenylen-Gruppe, welche durch Stickstoff, Schwefel, Sauerstoff oder -O-CO- unterbrochen sein kann bzw. diese enthalten kann.

B steht in den allgemeinen Formeln ganz besonders bevorzugt für Wasserstoff; für gegebenenfalls durch CN, Methoxy, Ethoxy, Isopropoxy, Methylthio, Ethylthio, Halogen, C₁-C₄-Alkylcarbonyloxy oder Phenyl substituierte Reste aus der Reihe C₁-C₆-Alkyl, C₃-C₆-Alkenyl, C₃-C₆-Alkinyl und C₃-C₆-Cycloalkyl oder für gegebenenfalls durch Halogen, CN, Nitro, C₁-C₄-Alkyl, C₁-C₄-Alkoxy, C₁-C₄-Alkylthio, C₁-C₄-Halogenalkyl und/oder C₁-C₄-Halogenalkoxy substituierte Reste aus der Reihe Phenyl, Phenyl-C₁-C₂-alkyl, Thienyl, Furyl, Thiazolyl, Pyridyl und Pyrazolyl; und
A und B in den allgemeinen Formeln bilden gemeinsam bevorzugt eine gegebenenfalls durch C₁-C₄-Alkyl, CF₃, CCl₃, C₂F₅, C₂Cl₂F₃, Methoxy, Ethoxy und/oder Halogen substituierte C₂-C₅-Alkylen- oder C₂-C₅-Alkenylen-Gruppe, welche durch Schwefel oder Sauerstoff unterbrochen sein kann bzw. diese enthalten kann.

In einer besonders hervorgehobenen Ausführungsform der vorliegenden Erfindung steht B für Wasserstoff, für gegebenenfalls durch Halogen, CN, Methoxy, Methylthio, substituiertes C₁-C₄-Alkyl oder für gegebenenfalls durch Halogen, Nitro, C₁-C₄-Alkyl und/oder C₁-C₄-Alkoxy substituierte Reste aus der Reihe Phenyl, Furyl, Thiazolyl und Pyridyl und A und B bilden gemeinsam die Gruppen -(CH₂)₃-, -(CH₂)₄-, -(CH₂)₅-, -CH₂-CH(CH₃)(CH₂)₂-, -CH₂O-(CH₂)₂- und -CH₂S(CH₂)₂-, die gegebenenfalls durch CF₃ oder OCH₃ substituiert sein können wie z.B. -(CH₂)₃-CHCl-, 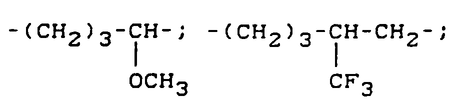 -C(CH₃)₂-O-C(CH₃)₂-, -CH₂-O-C(CH₃)₂-.

In den allgemeinen Formeln steht X bevorzugt für Halogen, C₁-C₆-Alkyl oder C₁-C₆-Alkoxy, besonders bevorzugt für Halogen, C₁-C₄-Alkyl oder C₁-C₄-Alkoxy und ganz besonders bevorzugt für Fluor, Chlor, Brom, Methyl, Ethyl, n- und i-Propyl, Methoxy oder Ethoxy und in einer besonders hervorgehobenen Ausführungsform der Erfindung für Methyl.

In den allgemeinen Formeln steht Y bevorzugt für Wasserstoff, Halogen, C₁-C₆-Alkyl, C₁-C₆-Alkoxy oder C₁-C₆-Halogenalkyl, besonders bevorzugt für Wasserstoff, Halogen, C₁-C₄-Alkyl, C₁-C₄-Halogenalkyl oder C₁-C₄-Alkoxy und ganz besonders bevorzugt für Wasserstoff, Fluor, Chlor, Brom, C₁-C₄-Alkyl oder Trifluormethyl und in einer besonders hervorgehobenen Ausführungsform der Erfindung für Methyl.

In den allgemeinen Formeln steht Z bevorzugt für Wasserstoff, Halogen, C₁-C₆-Alkyl oder C₁-C₆-Alkoxy, besonders bevorzugt für Wasserstoff, Halogen, C₁-C₄-Alkyl oder C₁-C₄-Alkoxy und ganz besonders bevorzugt für Wasserstoff, Fluor, Chlor, Brom, C₁-C₄-Alkyl, Methoxy oder Ethoxy und in einer besonderen Ausführungsform der Erfindung für Methyl (vorzugsweise in der 6-Position des Phenylringes).

In den allgemeinen Formeln steht n für 1, 2 oder 3, vorzugsweise für 1 oder 2 und ganz besonders bevorzugt für 1 (wobei sich Z vorzugsweise in der 6-Position des Phenylringes befindet).

G steht in den allgemeinen Formeln bevorzugt für Wasserstoff oder eine der Gruppen -COR³, 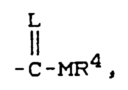 -SO₂-R⁵, 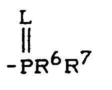 und -CONR⁸R⁹, besonders bevorzugt für Wasserstoff, -COR³, 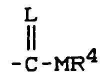 oder -CONR⁸R⁹ und ganz besonders bevorzugt für Wasserstoff, -COR³ oder 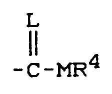 und in einer besonders hervorgehobenen Ausführungsform der Erfindung für Waserstoff.

R³ steht in den allgemeinen Formeln bevorzugt für gegebenenfalls durch Halogen substituierte Reste aus der Reihe C₁-C₂₀-Alkyl, C₂-C₂₀-Alkenyl, C₁-C₈-Alkoxy-C₁-C₈-alkyl, C₁-C₈-Alkylthio-C₁-C₈-alkyl, C₁-C₈-Polyalkoxy-C₁-C₈-alkyl und gegebenenfalls durch Sauerstoff und/oder Schwefel unterbrochenes C₃-C₈-Cycloalkyl; für gegebenenfalls durch Halogen, Nitro, C₁-C₆-Alkyl, C₁-C₆-Alkoxy, C₁-C₆-Halogenalkyl und/oder C₁-C₆-Halogenalkoxy substituiertes Phenyl; für gegebenenfalls durch Halogen, C₁-C₆-Alkyl, C₁-C₆-Alkoxy, C₁-C₆-Halogenalkyl und/oder C₁-C₆-Halogenalkoxy substituiertes Phenyl-C₁-C₆-alkyl; für gegebenenfalls durch Halogen und/oder C₁-C₆-Alkyl substituiertes Hetaryl; für gegebenenfalls durch Halogen und/oder C₁-C₆-Alkyl substituiertes Phenoxy-C₁-C₆-alkyl oder für gegebenenfalls durch Halogen, Amino und/oder C₁-C₆-Alkyl substituiertes Hetaryloxy-C₁-C₆-alkyl.

R³ steht in den allgemeinen Formeln besonders bevorzugt für gegebenenfalls durch Halogen substituierte Reste aus der Reihe C₁-C₁₆-Alkyl, C₂-C₁₆-Alkenyl, C₁-C₆-Alkoxy-C₁-C₆-alkyl, C₁-C₆-Alkylthio-C₁-C₆-alkyl, C₁-C₆-Polyalkoxy-C₁-C₆-alkyl und gegebenenfalls durch Sauerstoff und/oder Schwefel unterbrochenes C₃-C₇-Cycloalkyl; für gegebenenfalls durch Halogen, Nitro, C₁-C₄-Alkyl, C₁-C₄-Alkoxy, C₁-C₃-Halogenalkyl und/oder C₁-C₃-Halogenalkoxy substituiertes Phenyl; für gegebenenfalls durch Halogen, C₁-C₄-Alkyl, C₁-C₄-Alkoxy, C₁-C₃-Halogenalkyl und/oder C₁-C₃-Halogenalkoxy substituiertes Phenyl-C₁-C₄-alkyl; für gegebenenfalls durch Halogen und/oder C₁-C₆-Alkyl substituiertes Hetaryl; für gegebenenfalls durch Halogen und/oder C₁-C₄-Alkyl substituiertes Phenoxy-C₁-C₅-alkyl oder für gegebenenfalls durch Halogen, Amino und/oder C₁-C₄-Alkyl substituiertes Hetaryloxy-C₁-C₅-alkyl.

R³ steht in den allgemeinen Formeln ganz besonders bevorzugt für gegebenenfalls durch Fluor und/oder Chlor substituierte Reste aus der Reihe C₁-C₁₄-Alkyl, C₂-C₁₄-Alkenyl, C₁-C₄-Alkoxy-C₁-C₆-alkyl, C₁-C₄-Alkylthio-C₁-C₆-alkyl, C₁-C₄-Polyalkoxy-C₁-C₄-alkyl und gegebenenfalls durch 1 oder 2 Sauerstoff- und/oder Schwefelatome unterbrochenes C₃-C₆-Cycloalkyl; für gegebenenfalls durch Fluor, Chlor, Brom, Nitro, C₁-C₃-Alkyl, Methoxy, Ethoxy, Trifluormethyl und/oder Trifluormethoxy substituiertes Phenyl; für gegebenenfalls durch Fluor, Chlor, Brom, C₁-C₃-Alkyl, Methoxy, Ethoxy, Trifluormethyl und/oder Trifluormethoxy substituiertes Phenyl-C₁-C₃-alkyl; für gegebenenfalls durch Fluor, Chlor, Brom, Methyl und/oder Ethyl substituierte Reste aus der Reihe Pyridyl, Pyrimidyl, Thiazolyl und Pyrazolyl; für gegebenenfalls durch Fluor, Chlor, Methyl und/oder Ethyl substituiertes Phenoxy-C₁-C₄-alkyl oder für gegebenenfalls durch Fluor, Chlor, Amino, Methyl und/oder Ethyl substituierte Reste aus der Reihe Pyridyloxy-C₁-C₄-alkyl, Pyrimidyloxy-C₁-C₄-alkyl und Thiazolyloxy-C₁-C₄-alkyl.

In einer besonders hervorgehobenen Ausführungsform der vorliegenden Erfindung steht R³ für C₁-C₆-Alkyl oder C₁-C₆-Alkoxy.

R⁴ steht in den allgemeinen Formeln bevorzugt für gegebenenfalls durch Halogen substituierte Reste aus der Reihe C₁-C₂₀-Alkyl, C₂-C₂₀-Alkenyl, C₁-C₈-Alkoxy-C₁-C₈-alkyl, C₁-C₈-Polyalkoxy-C₁-C₈-alkyl oder für gegebenenfallsdurch Halogen, Nitro, C₁-C₆-Alkyl, C₁-C₆-Alkoxy und/oder C₁-C₆-Halogenalkyl substituierte Reste aus der Reihe Phenyl und Benzyl.

R⁴ steht in den allgemeinen Formeln besonders bevorzugt für gegebenenfalls durch Halogen substituierte Reste aus der Reihe C₁-C₁₆-Alkyl, C₂-C₁₆-Alkenyl, C₁-C₆-Alkoxy-C₁-C₆-alkyl, C₁-C₆-Polyalkoxy-C₁-C₆-alkyl oder für gegebenenfalls durch Halogen, Nitro, C₁-C₄-Alkyl, C₁-C₃-Alkoxy und/oder C₁-C₃-Halogenalkyl substituierte Reste aus der Reihe Phenyl und Benzyl.

R⁴ steht in den allgemeinen Formeln ganz besonders bevorzugt für gegebenenfalls durch Fluor und/oder Chlor substituierte Reste aus der Reihe C₁-C₁₄-Alkyl, C₂-C₁₄-Alkenyl, C₁-C₄-Alkoxy-C₁-C₆-alkyl, C₁-C₄-Polyalkoxy-C₁-C₆-alkyl oder für gegebenenfalls durch Fluor, Chlor, Nitro, C₁-C₃-Alkyl, Methoxy, Ethoxy und/oder Trifluormethyl substituierte Reste aus der Reihe Phenyl und Benzyl.

In einer besonders hervorgehobenen Ausführungsform der Erfindung steht R⁴ für C₁-C₆-Alkyl, welches durch Halogen substituiert sein kann.

R⁵, R⁶ und R⁷ stehen in den allgemeinen Formeln unabhängig voneinander bevorzugt für gegebenenfalls durch Halogen substituierte Reste aus der Reihe C₁-C₈-Alkyl, C₁-C₈-Alkoxy, C₁-C₈-Alkylthio, C₁-C₈-Alkylamino, Di-(C₁-C₈)-alkylamino, C₂-C₅-Alkenylthio, C₂-C₅-Alkinylthio, C₃-C₇-Cycloalkylthio oder für gegebenenfalls durch Halogen, Nitro, Cyano, C₁-C₄-Alkoxy, C₁-C₄-Alkylthio, C₁-C₄-Halogenalkoxy, C₁-C₄-Halogenalkylthio, C₁-C₄-Alkyl und/oder C₁-C₄-Halogenalkyl substituierte Reste aus der Reihe Phenyl, Phenyloxy und Phenylthio.

R⁵, R⁶ und R⁷ stehen in den allgemeinen Formeln unabhängig voneinander besonders bevorzugt für gegebenenfalls durch Halogen substituierte Reste aus der Reihe C₁-C₆-Alkyl, C₁-C₆-Alkoxy, C₁-C₆-Alkylthio, C₁-C₆-Alkylamino, Di-(C₁-C₆)-alkylamino, C₂-C₄-Alkenylthio, C₂-C₄-Alkinylthio, C₃-C₆-Cycloalkylthio oder für gegebenenfalls durch Halogen, Nitro, Cyano, C₁-C₃-Alkoxy, C₁-C₃-Alkylthio, C₁-C₃-Halogenalkoxy, C₁-C₃-Halogenalkylthio, C₁-C₃-Alkyl und/oder C₁-C₃-Halogenalkyl substituierte Reste aus der Reihe Phenyl, Phenyloxy und Phenylthio.

R⁵, R⁶ und R⁷ stehen in den allgemeinen Formeln unabhängig voneinander ganz besonders bevorzugt für gegebenenfalls durch Fluor und/oder Chlor substituierte Reste aus der Reihe C₁-C₄-Alkyl, C₁-C₄-Alkoxy, C₁-C₄-Alkylthio, C₁-C₄-Alkylamino oder Di-(C₁-C₄)-alkylamino, oder für gegebenenfalls durch Halogen, Nitro, Cyano, C₁-C₂-Alkoxy, C₁-C₂-Alkylthio, C₁-C₂-Fluoralkoxy, C₁-C₂-Chloralkoxy, C₁-C₂-Fluoralkylthio, C₁-C₂-Chloralkylthio und/oder C₁-C₃-Alkyl substituierte Reste aus der Reihe Phenyl,Phenyloxy und Phenylthio.

In einer besonders hervorgehobenen Ausführungsform der Erfindung stehen R⁵, R⁶ und R⁷ unabhängig voneinander für gegebenenfalls durch Halogen substituiertes C₁-C₆-Alkyl.

R⁸ und R⁹ stehen in den allgemeinen Formeln unabhängig voneinander bevorzugt für Wasserstoff, für gegebenenfalls durch Halogen substituierte Reste aus der Reihe C₁-C₂₀-Alkyl, C₁-C₂₀-Alkoxy, C₃-C₁₀-Cycloalkyl, C₂-C₈-Alkenyl, C₁-C₈-Alkoxy-C₁-C₈-alkyl oder für gegebenenfalls durch Halogen, C₁-C₂₀-Alkyl, C₁-C₂₀-Halogenalkyl und/oder C₁-C₂₀-Alkoxy substituierte Reste aus der Reihe Phenyl und Benzyl oder R⁸ und R⁹ bilden gemeinsam eine C₂-C₆-Alkylen-Gruppe, welche durch Sauerstoff und/oder Schwefel unterbrochen sein kann.

R⁸ und R⁹ stehen in den allgemeinen Formeln unabhängig voneinander besonders bevorzugt für Wasserstoff, für gegebenenfalls durch Halogen substituierte Reste aus der Reihe C₁-C₁₆-Alkyl, C₁-C₁₆-Alkoxy, C₃-C₁₆-Cycloalkyl, C₂-C₆-Alkenyl, C₁-C₆-Alkoxy-C₁-C₆-alkyl oder für gegebenenfalls durch Halogen, C₁-C₆-Alkyl, C₁-C₆-Halogenalkyl und/oder C₁-C₆-Alkoxy substituierte Reste aus der Reihe Phenyl und Benzyl oder R⁸ und R⁹ bilden gemeinsam eine C₂-C₆-Alkylen-Gruppe, welche durch Sauerstoff und/oder Schwefel unterbrochen sein kann,

R⁸ und R⁹ stehen in den allgemeinen Formeln unabhängig voneinander ganz besonders bevorzugt für Wasserstoff, für gegebenenfalls durch Halogen substituierte Reste aus der Reihe C₁-C₁₀-Alkyl, C₁-C₁₀-Alkoxy, C₁-C₁₀-Alkoxy-C₁-C₁₀-alkyl oder für gegebenenfalls durch Halogen, C₁-C₄-Alkyl, C₁-C₄-Halogenalkyl und/oder C₁-C₄-Alkoxy substituierte Reste aus der Reihe Phenyl und Benzyl oder R⁸ und R⁹ bilden gemeinsam eine C₂-C₄-Alkylen-Gruppe, welche durch Sauerstoff und/oder Schwefel unterbrochen sein kann.

In einer besonders hervorgehobenen Ausführungsform der Erfindung stehen R⁸ und R⁹ unabhängig voneinander für Wasserstoff oder gegebenenfalls durch Halogen substituiertes C₁-C₆-Alkyl.

L steht in den allgemeinen Formeln bevorzugt (sowie besonders bevorzugt und ganz besonders bevorzugt) für Sauerstoff.

M steht in den allgemeinen Formeln bevorzugt (sowie besonders bevorzugt und ganz besonders bevorzugt) für Sauerstoff.

Die oben aufgeführten allgemeinen oder in Vorzugsbereichen aufgeführten Restedefinitionen bzw. Erläuterungen können untereinander, also auch zwischen den jeweiligen Vorzugsbereichen beliebig kombiniert werden. Sie gelten für die Endprodukte sowie für die Vor- und Zwischenprodukte entsprechend.

Erfindungsgemäß bevorzugt werden die Verbindungen der allgemeinen Formel (I), in welchen eine Kombination der vorstehend als bevorzugt aufgeführten Bedeutungen vorliegt.

Erfindungsgemäß besonders bevorzugt werden die Verbindungen der allgemeinen Formel (I), in welchen eine Kombination der vorstehend als besonders bevorzugt aufgeführten Bedeutungen vorliegt.

Erfindungsgemäß ganz besonders bevorzugt werden die Verbindungen der allgemeinen Formel (I) verwendet, in welchen eine Kombination dieser vorstehend als ganz besonders bevorzugt aufgeführten Bedeutungen vorliegt.

Die zur Durchführung der erfindungsgemäßen Verfahrensvariante b), c), d), e), f), g) und h) als Ausgangsstoffe benötigten 3-Aryl-pyrone sind nach Verfahrensvariante a) erhältlich.

Die zur Durchführung der erfindungsgemäßen Verfahrensvarianten b), c), d), e), f), g) und h) außerdem als Ausgangsstoffe benötigten Säurehalogenide der Formel (IV), Carbonsäureanhydride der Formel (V), Chlorameisensäureester oder Chlorameisensäurethioester der Formel (VI), Chlormonothioameisensäureester oder Chlordithioameisensäureester der Formel (VII), Alkylhalogenide der Formel (VIII), Sulfonsäurechloride der Formel (IX), Phosphorverbindungen der Formel (X), die Verbindungen der Formeln (XI) und (XII) sowie die eingesetzten Metallhydroxide und Amine sind allgemein bekannte Verbindungen der organischen bzw. anorganischen Chemie.

Auch die Carbonylverbindungen der Formel (II) sind allgemein bekannte Verbindungen in der organischen Chemie.

Die Verbindungen der Formel (III) sind teilweise bekannt (vgl. beispielsweise Org. Prep. Proced.Int., 7(4), 155-8, 1975 und DE 19 45 703). Die noch nicht bekannten Verbindungen lassen sich aber nach im Prinzip bekannten Methoden in einfacher Weise analog herstellen. So erhält man z.B. Halogencarbonylketone der Formel (III), wenn man Arylmalonsäuren der Formel (XIII) 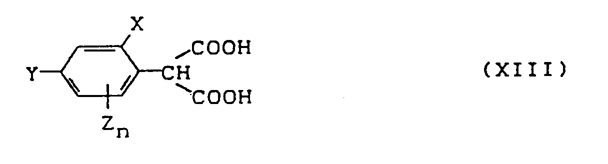 in welcher
X, Y, Z und n die oben angegebene Bedeutung haben,
mit Säurehalogeniden, wie beispielsweise Thionylchlorid, Phosphor(V)chlorid, Phosphor(III)chlorid, Oxalylchlorid, Phosgen oder Thionylbromid gegebenenfalls in Gegenwart von Katalysatoren, wie beispielsweise Diethylformamid, Methl-Sterylformamid oder Triphenylphosphin, umsetzt.

Die Arylmalonsäuren der Formel (XIII) sind allgemein bekannte Verbindungen der organischen Chemie (vgl. z.B. Organikum, VEB Deutscher Verlag der Wissenschaften, Berlin 1977, S.517 ff).

Als Verdünnungsmittel können bei der erfindungsgemäßen Verfahrensvariante a) alle inerten organischen Solventien eingesetzt werden. Vorzugsweise verwendbar sind Kohlenwasserstoffe, wie Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid und N-Methyl-pyrrolidon.

Als Säureakzeptoren können bei der Durchführung der erfindungsgemäßen Verfahrensvariante a) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung der erfindungsgemäßen Verfahrensvariante a) innerhalb eines größeren Bereiches variiert werden. Zweckmäßigerweise arbeitet man bei Temperaturen zwischen 0°C und 250°C, vorzugsweise zwischen 50°C und 220°C.

Die erfindungsgemäße Verfahrensvariante a) wird zweckmäßigerweise unter Normaldruck durchgeführt.

Bei der Durchführung der erfindungsgemäßen Verfahrensvariante a) setzt man die Reaktionskomponenten der Formeln (II) und (III) und gegebenenfalls die Säureakzeptoren zweckmäßigerweise in etwa äquimolaren Mengen ein. Es ist jedoch auch möglich, die eine oder andere Komponente in einem größeren Überschuß (bis zu 5 Mol) zu verwenden.

Als Verdünnungsmittel können bei der erfindungsgemäßen Verfahrensvariante b) α) bei Verwendung der Säurehalogenide alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Verwendet man die entsprechenden Carbonsäurehalogenide so kommen als Säurebindemittel bei der Umsetzung nach der erfindungsgemäßen Verfahrensvariante b) α) alle üblichen Säureakzeptoren in Betracht. Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide, wie Natrium- und Kaliumhydroxid.

Die Reaktionstemperaturen können auch bei der erfindungsgemäßen Verfahrensvariante b) α) innerhalb eines größeren Bereiches variiert werden. Zweckmäßigerweise arbeitet man bei Temperaturen zwischen -20°C und +150°C, vorzugsweise zwischen 0°C und 100°C.

Bei der Durchführung der erfindungsgemäßen Verfahrensvariante b) α) werden die Ausgangsstoffe der Formeln (I) und das Carbonsäurehalogenid der Formel (V) vorzugsweise in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Verwendet man bei der erfindungsgemäßen Verfahrensvariante b) β) als Reaktionskomponente der Formel (V) Carbonsäureanhydride, so können als Verdünnungsmittel vorzugsweise diejenigen Verdünnungsmittel verwendet werden, die auch bei der Verwendung von Säurehalogeniden vorzugsweise in Betracht kommen, Im übrigen kann auch ein im Überschuß eingesetztes Carbonsäurehydrid gleichzeitig als Verdünnungsmittel fungieren. Auch als Säureakzeptoren können die bei der Verfahrensvariante b) α) angegebenen Säureakzeptoren eingesetzt werden,

Die Reaktionstemperaturen können bei der erfindungsgemäßen Verfahrensvariante b) β) auch bei der Verwendung von Carbonsäureanhydriden innerhalb eines größeren Bereiches variiert werden. Zweckmäßigerweise arbeitet man bei Temperaturen zwischen -20°C und +150°C, vorzugsweise zwischen 0°C und 100°C.

Bei der Durchführung des erfindungsgemäßen Verfahrens werden die Ausgangsstoffe der Formel (I) und das Carbonsäureanhydrid der Formel (V) vorzugsweise in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Zweckmäßigerweise geht man so vor, daß man Verdünnungsmitel und im Überschuß vorhandenes Carbonsäureanhydrid sowie die entstehende Carbonsäure durch Destillation oder durch Waschen mit einem organischen Lösungsmittel oder mit Wasser entfernt.

Verwendet man bei der Verfahrensvariante c) die entsprechenden Chlorameisensäureester bzw. Chlorameisensäurethiolester so kommen als Säurebindemittel bei der Umsetzung alle üblichen Säureakzeptoren in Betracht.

Vorzugsweise verwendbar sind tertiäre Amine, wie Triethylamin, Pyridin, 1,4-Diazabicyclo[2.2.2]octan (DABCO), 1,8-Diazabicyclo[5.4.0]undec-7-en (DBU), DBA, Hünig-Base und N,N-Dimethyl-anilin, ferner Erdalkalimetalloxide, wie Magnesium- und Calcium-oxid, außerdem Alkali- und Erdalkalimetall-carbonate, wie Natriumcarbonat, Kaliumcarbonat und Calciumcarbonat sowie Alkalihydroxide, wie Natrium- oder Kaliumhydroxid.

Als Verdünnungsmittel können bei der erfindungsgemäßen Verfahrensvariante c) bei Verwendung der Chlorameisensäureester bzw. Chlorameisensäurethiolester alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Bei Verwendung der Chlorameisensäureester bzw. Chlorameisensäurethiolester als Carbonsäure-Derivate der Formel (VI) können die Reaktionstemperaturen bei der Durchführung der erfindungsgemäßen Verfahrensvariante c) innerhalb eines größeren Bereiches variiert werden, Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Die erfindungsgemäße Verfahrensvariante c) wird zweckmäßigerweise unter Normaldruck durchgeführt.

Bei der Durchführung der erfindungsgemäßen Verfahrensvariante c) werden die Ausgangsstoffe der Formel (I) und der entsprechende Chlorameisensäureester bzw. Chlorameisensäurethiolester der Formel (VI) vorzugsweise in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Zweckmäßigerweise geht man so vor, daß man ausgefallene Salze entfernt und das verbleibende Reaktionsgemisch durch Abziehen des Verdünnungsmittels einengt.

Bei der Verfahrensvariante d) α) setzt man pro Mol Ausgangsverbindung der Formel (I) ca. 1 Mol Chlormonothioameisensäureester bzw. Chlordithioameisensäureester der Formel (VII) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindung (I) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Bei der Verfahrensvariante d) β) setzt man pro Mol Ausgangsverbindung der Formel (I) die äquimolare Menge bzw. einen Überschuß Schwefelkohlenstoff zu. Man arbeitet hierbei vorzugsweise bei Temperaturen von 0 bis 50°C und insbesondere bei 20 bis 30°C.

Oft ist es zweckmäßig zunächst aus der Verbindung der Formel (I) durch Zusatz eines Deprotonierungsmittels (wie z.B. Kaliumtertiärbutylat oder Natriumhydrid) das entsprechende Salz herzustellen. Man setzt die Verbindung (I) solange mit Schwefelkohlenstoff um, bis die Bildung der Zwischenverbindung abgeschlossen ist, z.B. nach mehrstündigem Rühren bei Raumtemperatur.

Die weitere Umsetzung mit dem Alkylhalogenid der Formel (VIII) erfolgt vorzugsweise bei 0 bis 70°C und insbesondere bei 20 bis 50°C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Bei der Verfahrensvariante e) setzt man pro Mol Ausgangsverbindung der Formel (I) ca. 1 Mol Sulfonsäurechlorid (IX) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Nitrile, Alkohole, Sulfone, Sulfoxide oder halogenierte Kohlenwasserstoffe wie Methylenchlorid.

Vorzugsweise werden Dimethylsulfoxid, Tetrahydrofuran und Dimethylformamid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Bei der Verfahrensvariante e) kann gegebenenfalls unter Phasen-Transfer-Bedingungen gearbeitet werden (W.J. Spillane et al.; J. Chem. Soc., Perkin Trans I, (3) 677-9 (1982)). In diesem Fall setzt man pro Mol Ausgangsverbindung der Formel (I) 0,3 bis 1,5 Mol Sulfonsäurechlorid (IX), bevorzugt 0,5 Mol bei 0° bis 150°C, vorzugsweise bei 20 bis 70°C um.

Als Phasen-Transfer-Katalysatoren können alle quartären Ammoniumsalze verwendet werden, vorzugsweise Tetraoctylammoniumbromid und Benzyltriethylammoniumchlorid. Als organische Lösungsmittel können in diesem Fall alle unpolaren inerten Lösungsmittel dienen, bevorzugt werden Benzol und Toluol eingesetzt.

Bei der Verfahrensvariante f) setzt man auf 1 Mol der Verbindung (I) 1 bis 2, vorzugsweise 1 bis 1,3 Mol der Phosphorverbindung der Formel (X) bei Temperaturen zwischen -40°C und 150°C, vorzugsweise zwischen -10 und 110°C um.

Als gegebenenfalls zugesetzte Verdünnungsmittel kommen alle inerten, polaren organischen Lösungsmittel in Frage wie Ether, Amide, Nitrile, Alkohole, Sulfide, Sulfone, Sulfoxide etc.

Vorzugsweise werden Acetnitril, Dimethylsulfoxid, Tetrahydrofuran und Dimethylformamid eingesetzt.

Als gegebenenfalls zugesetzte Säurebindemittel kommen übliche anorganische oder organische Basen in Frage wie Hydroxide, Carbonate. Beispielhaft seien Natriumhydroxid, Natriumcarbonat, Kaliumcarbonat, Pyridin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Die Verfahrensvariante g) ist dadurch gekennzeichnet, daß man Verbindungen der Formel (I) mit Metallhydroxiden oder Aminen umsetzt.

Als Verdünnungsmittel können bei dem erfindungsgemäßen Verfahren vorzugsweise Ether wie Tetrahydrofuran, Dioxan, Diethylether oder aber Alkohole wie Methanol, Ethanol, Isopropanol, aber auch Wasser eingesetzt werden, Das Verfahren wird vorzugsweise unter Normaldruck durchgeführt. Die Reaktionstemperaturen liegen im allgemeinen zwischen -20°C und 100°C, vorzugsweise zwischen 0°C und 50°C.

Bei der Durchführung der erfindungsgemäßen Verfahrensvariante g) werden die Ausgangsstoffe im allgemeinen in angenähert äquimolaren Mengen verwendet. Es ist jedoch auch möglich, die eine oder andere Komponente in einem größeren Überschuß (bis zu 2 Mol) einzusetzen. Zweckmäßigerweise geht man so vor, daß man das Reaktionsgemisch durch Abziehen des Verdünnungsmittels einengt.

Bei der Verfahrensvariante h) α) setzt man pro Mol Ausgangsverbindung der Formel (I) ca. 1 Mol Isocyanat der Formel (XI) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Bei der Verfahrensvariante h) β) setzt man pro Mol Ausgangsverbindung der Formel (I) ca. 1 Mol Carbamidsäurechlorid bzw. Thiocarbamidsäurechlorid der Formel (XII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Alkohole, Sulfone, Sulfoxide.

Vorzugsweise werden Dimethylsulfoxid, Tetrahydrofuran und Dimethylformamid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die erfindungsgemäßen Verbindungen der Formel I können zur Schädlingsbekämpfung eingesetzt werden. Schädlinge sind unerwünschte tierische Schädlinge, insbesondere Insekten, Milben und Nematoden, welche Pflanzen oder höhere Tiere schädigen. Zu den Schädlingen gehören jedoch auch unerwünschte Pflanzen.

Die erfindungsgemäßen Wirkstoffe eignen sich bei guter Pflanzenverträglichkeit und günstiger Wärmeblütertoxizität zur Bekämpfung von tierischen Schädlingen, vorzugsweise von Anthropoden, insbesondere von Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.

Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Daneben besitzen die erfindungsgemäßen Wirkstoffe der Formel (I) auch eine gute fungizide Wirksamkeit und lassen sich zur Bekämpfung von Pflanzenkrankheiten wie beispielsweise gegen den Erreger der Reisfleckenkrankheit (Pyricularia oryzae) einsetzen.

Die erfindungsgemäßen Wirkstoffe können zur Verwendung als Insektizide, Akarizide und Nematizide in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die erfindungsgemäßen Verbindungen eignen sich auch in besonderer Weise zur Behandlung von vegativem und generativem Vermehrungsmaterial, wie z.B. von Saatgut von Getreide, Mais, Gemüse u.s.w. oder von Zwiebeln, Stecklingen u.s.w.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe können auch als Herbizide, vorzugsweise als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen Unkräutern in dikotylen Kulturen sowohl im Vorauflauf- als auch im Nachauflauf-Verfahren.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinine, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streu en.

Die erfindungsgemäßen Wirkstoffe können sowohl vor, als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Zur Herstellung der Schädlingsbekämpfungsmittel können die erfindungsgemäßen Wirkstoffe in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmneben-Formulierungen.

Die Wirkstoffe Können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff
und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es Können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive Können mineralische und vegetabile Öle sein.

Es Können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe Können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Mittel enthalten bevorzugt neben wenigstens einer verbindung der allgmeeinen Formel (I) und gegebenenfalls neben erheblichen Streck- und Hilfsmitteln wenigstens einen oberflächenaktiven Stoff.

Die Wirkstoffe eignen sich bei günstiger Warmblütertoxizität auch zur Bekämpfung von tierischen Schädlingen (Ekto- und Endoparasiten) wie Arthropoden, vorzugsweise Insekten und Spinnentieren (Ektoparasiten), Cestoden, Trematoden, Nematoden und Acantocephalen (Endoparasiten), die in der Tierhaltung und Tierzucht bei Haus- und Nutztieren sowie Zoo-, Labor-, Versuchs- und Hobbytieren vorkommen. Sie sind dabei gegen alle oder einzelne Entwicklungsstadien der Schädlinge sowie gegen resistente und normal sensible Arten der Schädlinge wirksam.

Durch die Bekämpfung der tierischen Schädlinge sollen Krankheiten und deren Übertragung, Todesfälle und Leistungsminderungen (z.B. bei der Produktion von Fleisch, Milch, Wolle, Häuten, Eiern) verhindert werden, so daß durch den Einsatz der Wirkstoffe eine wirtschaftlichere und einfachere Tierhaltung möglich ist bzw. in bestimmten Gebieten erst möglich wird.

Zu den Schädlingen gehören:
Aus der Ordnung der Anoplura z.B. Haematopinus spp., Linognathus spp., Solenopotes spp., Pediculus spp., Pthirus spp.;
aus der Ordnung der Mallophaga z.B. Trimenopon spp., Menopon spp., Eomenacanthus spp., Menacanthus spp., Trichodectes spp., Felicola spp., Damalinea spp., Bovicola spp;
aus der Ordnung der Diptera z.B. Chrysops spp., Tabanus spp., Musca spp., Hydrotaea spp., Muscina spp., Haematobosca spp., Haematobia spp., Stomoxys spp., Fannia spp., Glossina spp., Lucilia spp., Calliphora spp., Auchmeromyia spp., Cordylobia spp., Cochliomyia spp., Chrysomyia spp., Sarcophaga spp., Wohlfartia spp., Gasterophilus spp., Oesteromyia spp,, Oedemagena spp., Hypoderma spp., Oestrus spp., Rhinoestrus spp., Melophagus spp., Hippobosca spp..

Aus der Ordnung der Siphonaptera z.B. Ctenocephalides spp., Echidnophaga spp., Ceratophyllus spp..

Aus der Ordnung der Metastigmata z.B. Hyalomma spp., Rhipicephalus spp., Boophilus spp., Amblyomma spp., Haemophysalis spp., Dermacentor spp., Ixodes spp., Argas spp., Ornithodorus spp., Otobius spp.;
aus der Ordnung der Mesastigmata z.B. Dermanyssus spp., Ornithonyssus spp., Pneumonyssus spp..

Aus der Ordnung der Prostigmata z.B. Cheyletiella spp., Psorergates spp., Myobia spp., Demodex spp., Neotrombicula spp.;
aus der Ordnung der Astigmata z.B. Acarus spp., Myocoptes spp., Psoroptes spp., Chorioptes spp., Otodectes spp., Sarcoptes spp., Notoedres spp., Knemidocoptes spp., Neoknemidocoptes spp. Lytodites spp., Laminosioptes spp..

Zu den Endoparasiten gehören:
Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegel, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung Kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspension zur oralen oder dermalen Anwendung sowie zur Injektion; Halbfeste Zubereitungen;
Formulierungen bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff entweder die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt;
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether; ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die vorliegende Erfindung betrifft somit auch die Verbindungen der allgemeinen Formel (I) zur Verwendung als Ekto- und Endoparasitizide sowie die Verwendung der Verbindungen der allgemeinen Formel (I) zur Herstellung eines Mittels zur Bekämpfung von Ekto- und Endoparasiten.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 5 bis 50 Gewichtsprozent.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

Die erfindungsgemäßen Verbindungen werden bevorzugt als Arthropodizie und Herbizide in den Bereichen Pflanzenschutz, Haushalt- und Hygiene sowie im Vorratsschutz, ganz besonders bevorzugt im Pflanzenschutz eingesetzt.

Wo nicht anderes angegeben wird sind alle Prozentangaben Gewichtsprozente.

Die Herstellung der erfindungsgemäßen Verbindungen der allgemeinen Formel (I) soll durch die folgenden Herstellungsbeispiele und die biologische Wirksamkeit durch die folgenden biologischen Beispiele erläutert werden.

### Beispiel 1

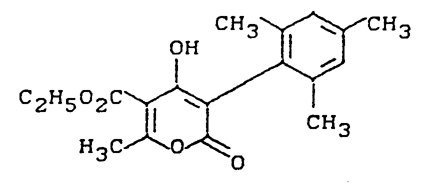

Zu 22,2 g (0,1 Mol) Chlorcarbonyl-2,4,6-trimethyl-phenyl-keten in 200 ml absolutem Toluol tropft man unter Feuchtigkeitsausschluß 13,0 g (0,1 Mol) Acetessigsäureethylester bei 20°C und erhitzt darauf 3 Stunden unter Rückfluß. Die Toluolphase wird mit Wasser gewaschen, das Lösungsmittel abdestilliert und der Rückstand an 1 kg Kieselgel (35 bis 70 µm) mit Toluol/Aceton (20:1 Volumenteile) chromatographiert. Man erhält 17,9 g 5-Ethoxy-4-hydroxy-6-methyl-3-(2,4,6-trimethyl-phenyl)-pyron (Ausbeute: 57 % der Theorie).
¹H-NMR (CDCl₃, TMS interner Standard): δ = 2,23 (s, 3H), 2,70 (s, 3H), 6,90 (s, 2H) und 11,75 (s, 1H).

### Beispiel 2

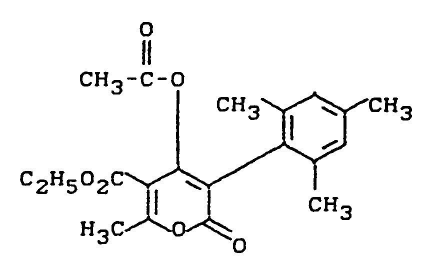

Zu 3,2 g (10 mmol) 5-Ethoxycarbonyl-4-hydroxy-6-methyl-3-(2,4,6-trimethylphenyl)-pyron in ml Ethylacetat tropft man bei 0°C 1,0 g (10 mmol) Triethylamin und darauf 0,8 g (10 mmol) Acetylchlorid. Man rührt 20 Stunden bei 20°C, filtriert, wäscht die organische Phase mit halbkonzentrierter Kochsalzlösung, trocknet über Natriumsulfat und dampft das Lösungsmittel im Vakuum ab, Der Rückstand wird an Kieselgel (35-70 µm) mit Toluol/Aceton (40:1 Volumenteile) chromatographiert. Man erhält 2,9 g 4-Acetoxy-5-ethoxycarbonyl-6-methyl-3-(2,4,6-trimethyl-phenyl)-pyron (Ausbeute: 81 % der Theorie), Fp. = 107 bis 109°C.

### Beispiel 3

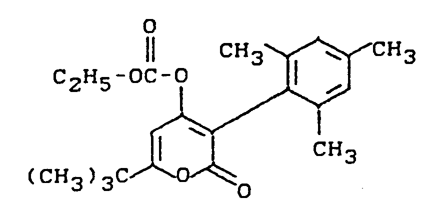

Zu 5,7 g (20 mmol) 4-Hydroxy-6-tert.-butyl-3-(2,4,6-trimethyl-phenyl)-pyron in 50 ml Ethylacetat tropft man 2,0 g (20 mmol) Triethylamin und darauf 2,2 g (20 mmol) Chlorameisensäureethylester in 20 ml Ethylacetat bei 0°C. Man rührt 20 Stunden bei 20°C, filtriert, wäscht die organische Phase mit halbkonzentrierter Kochsalzlösung, trocknet über Natriumsulfat und dampft das Lösungsmittel im Vakuum ab. DerRückstand wird an Kieselgel (35-70 µm) mit Toluol/Aceton (20:1 Volumenteile) chromatographiert. Man erhält 6,0 g 4-Ethoxycarbonyloxy-6-tert.-butyl-3-(2,4,6-trimethylphenyl)-pyron (Ausbeute: 84 % der Theorie). Fp. = 92-94°C.

In entsprechender Weise zu den Herstellungsbeispielen und gemäß den allgemeinen Angaben der Beschreibung zur Herstellung erhält man die in den nachfolgenden Tabellen 1 bis 3 formelmäßig aufgeführten 3-Arylpyron-Derivate der allgemeinen Formel (I).

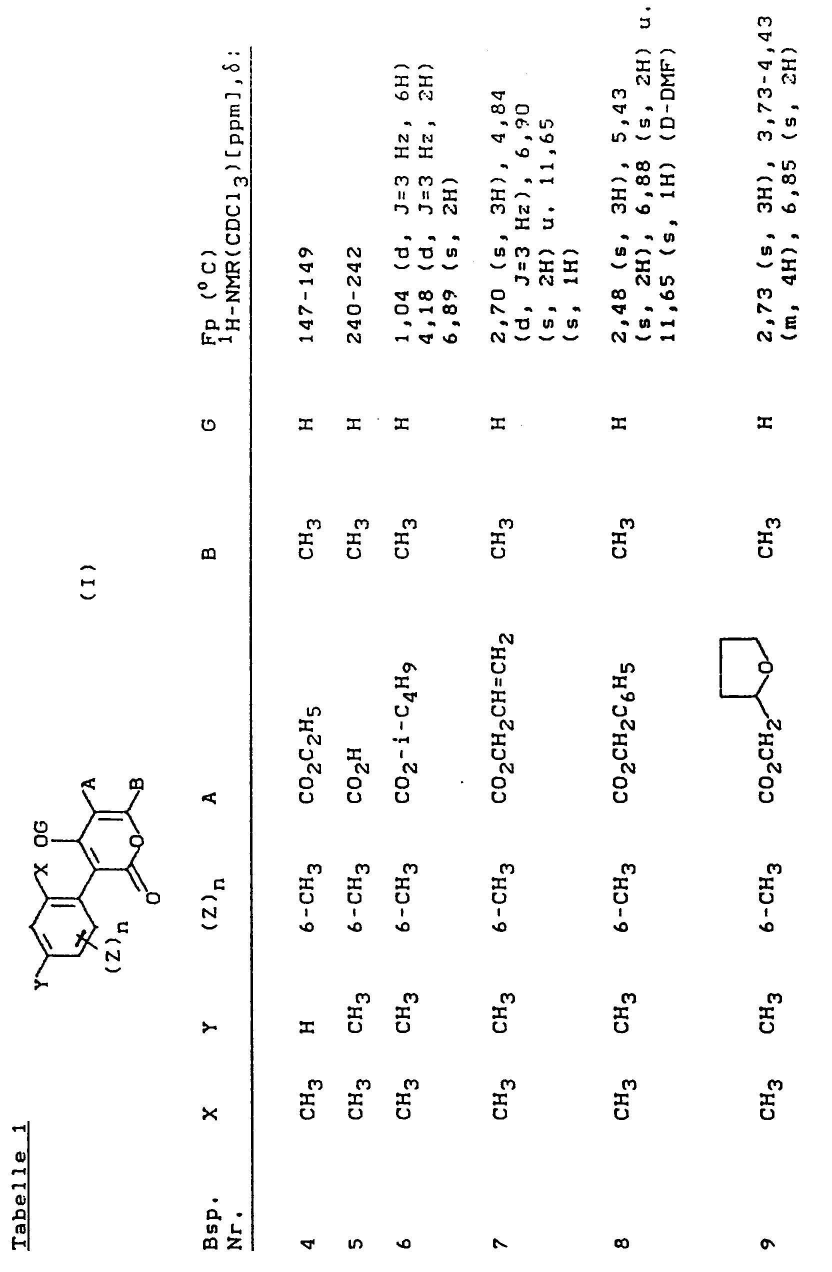

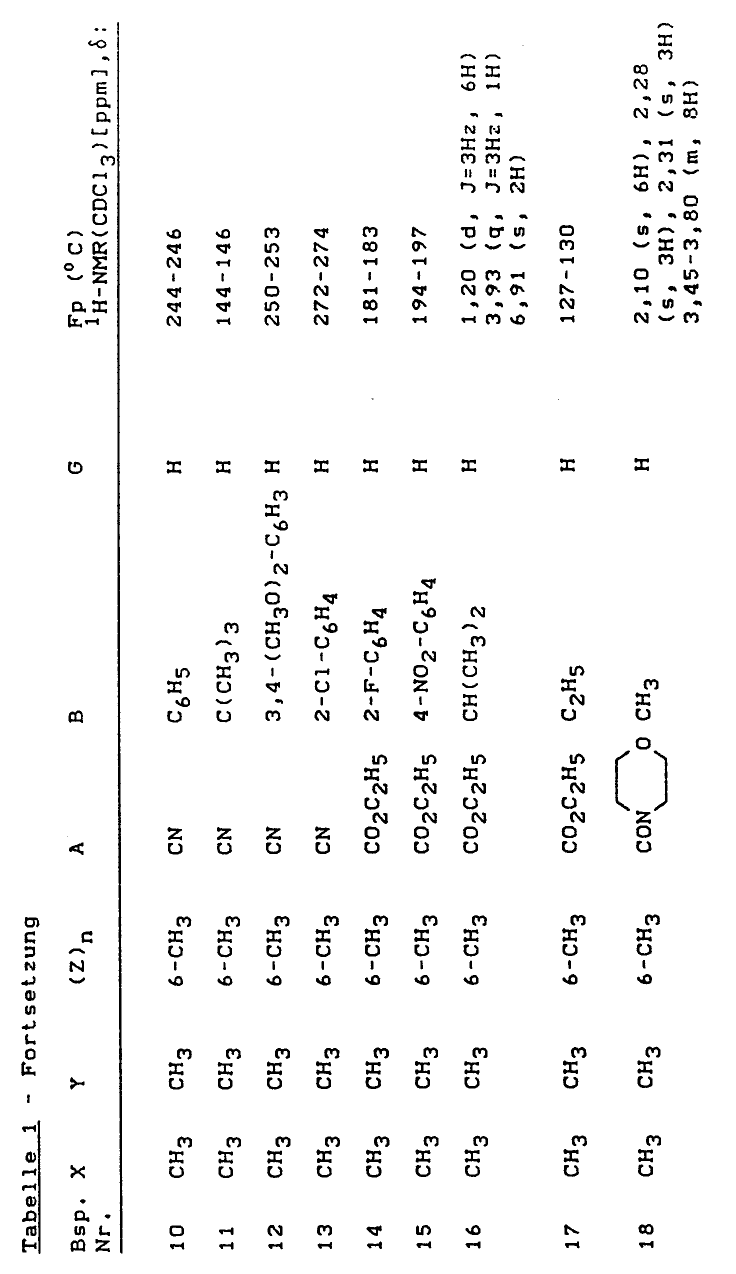

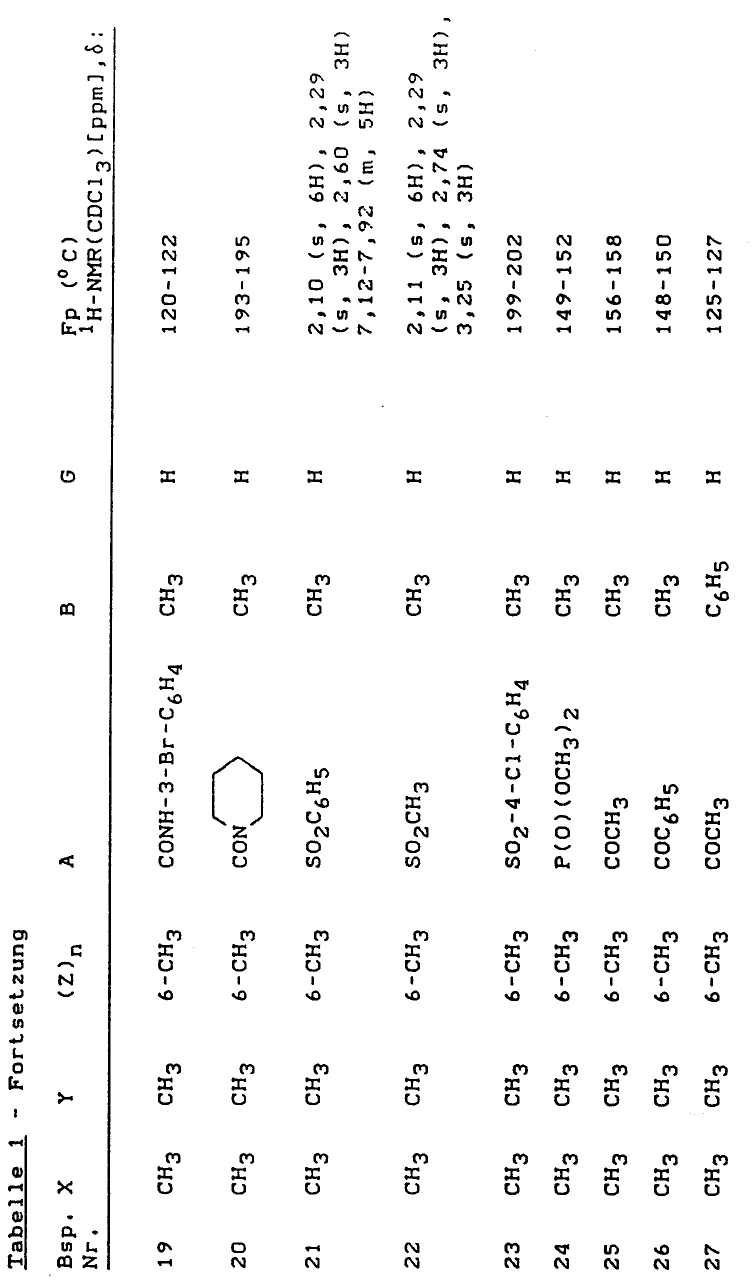

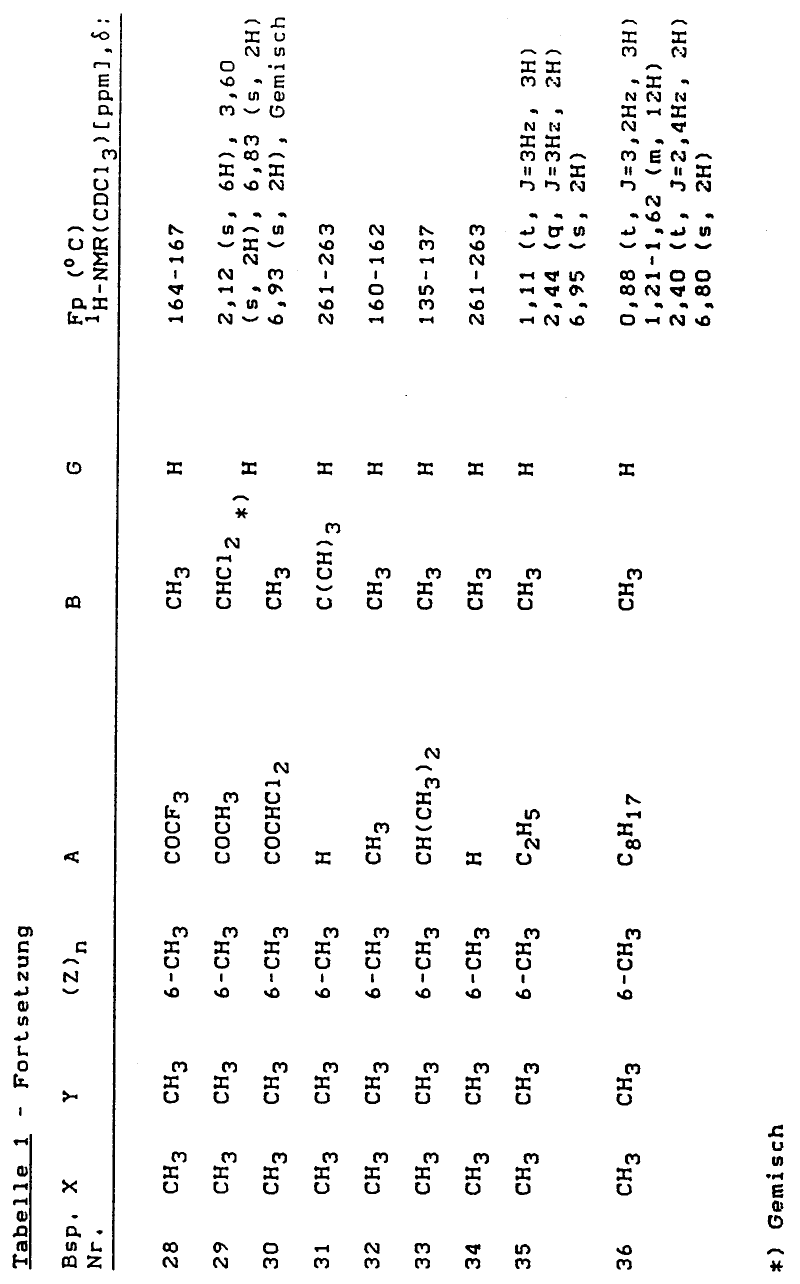

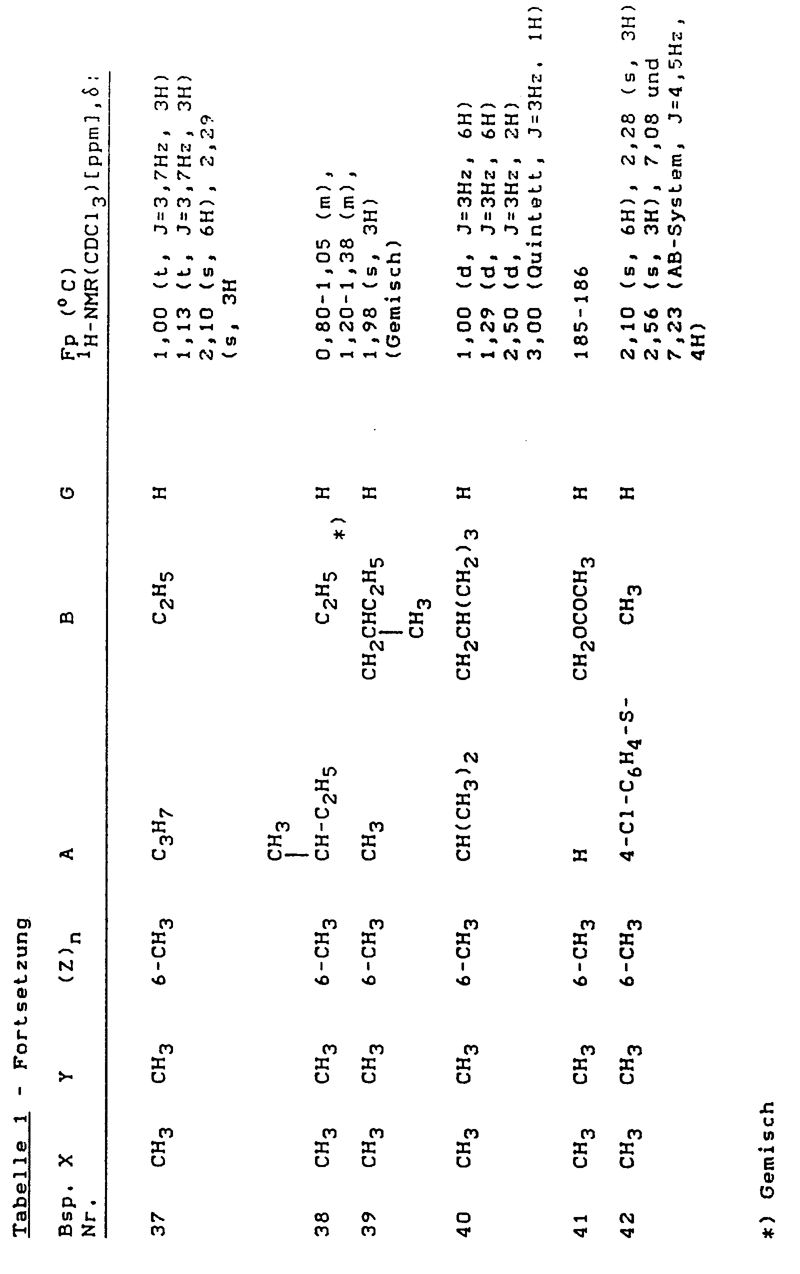

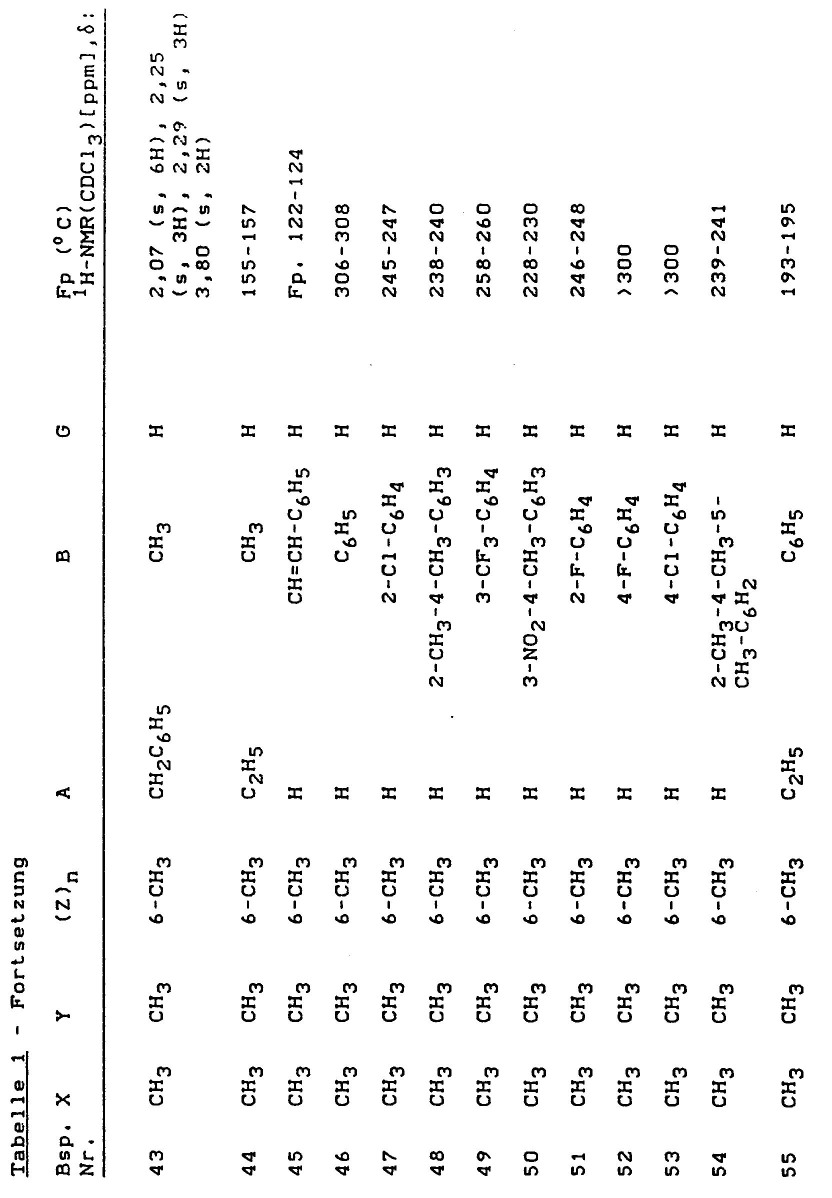

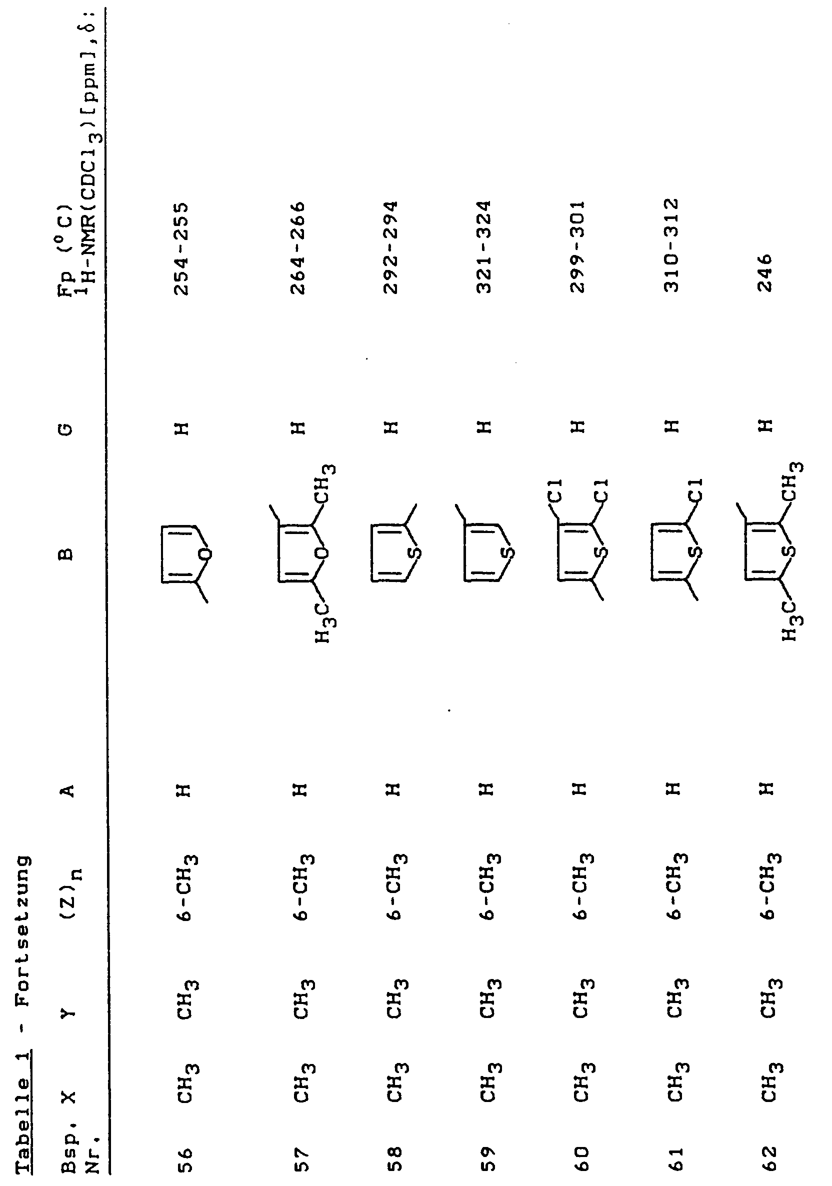

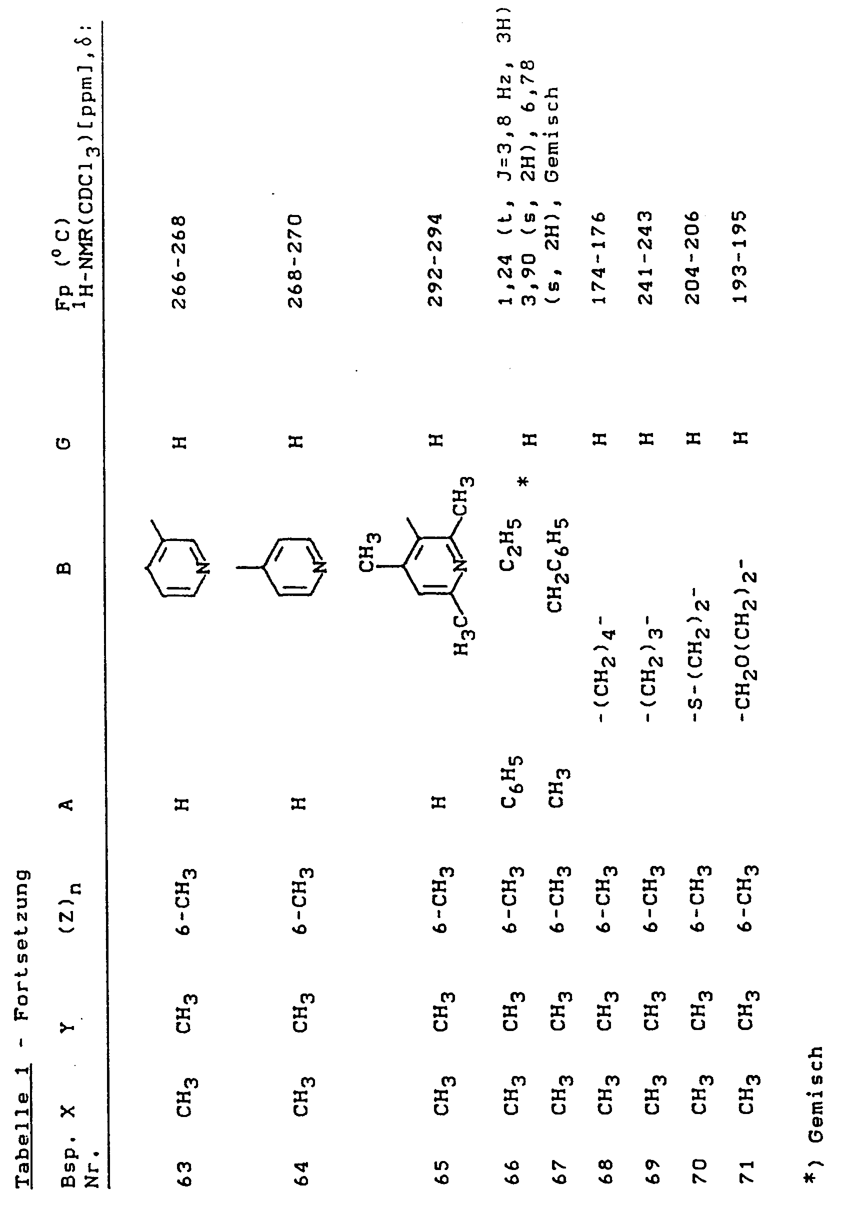

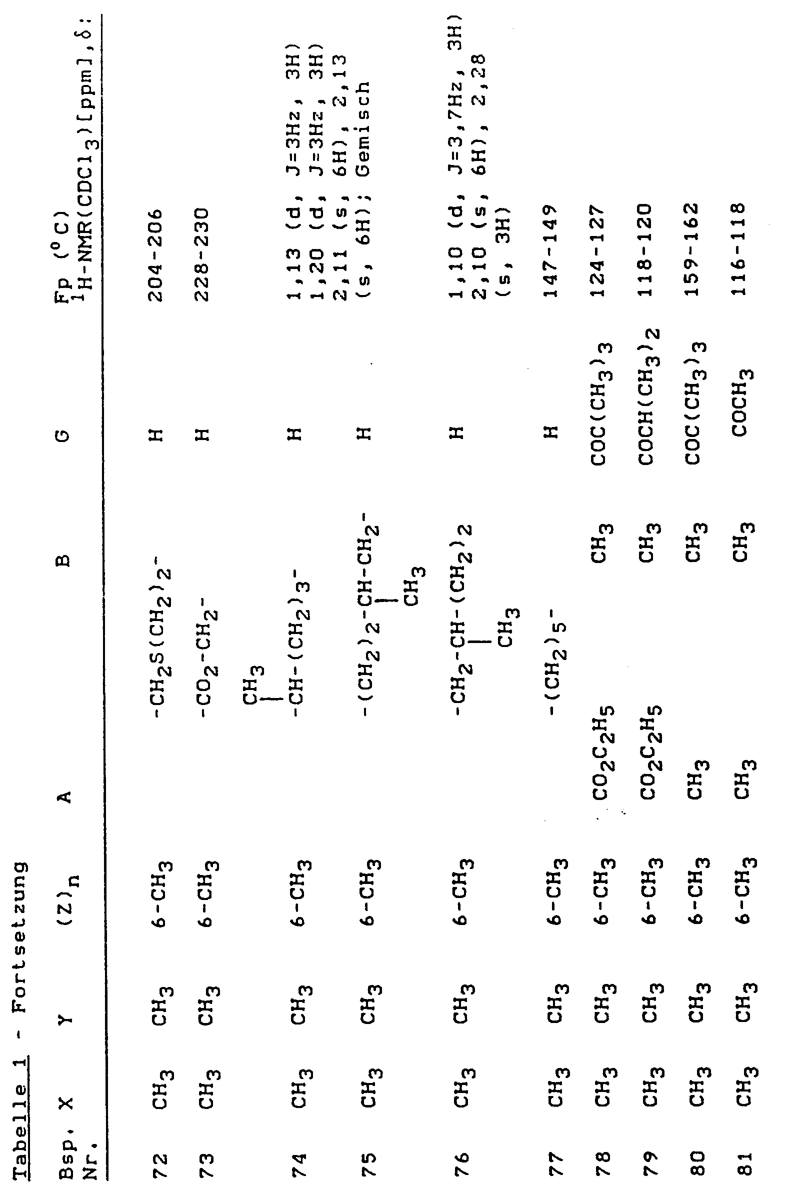

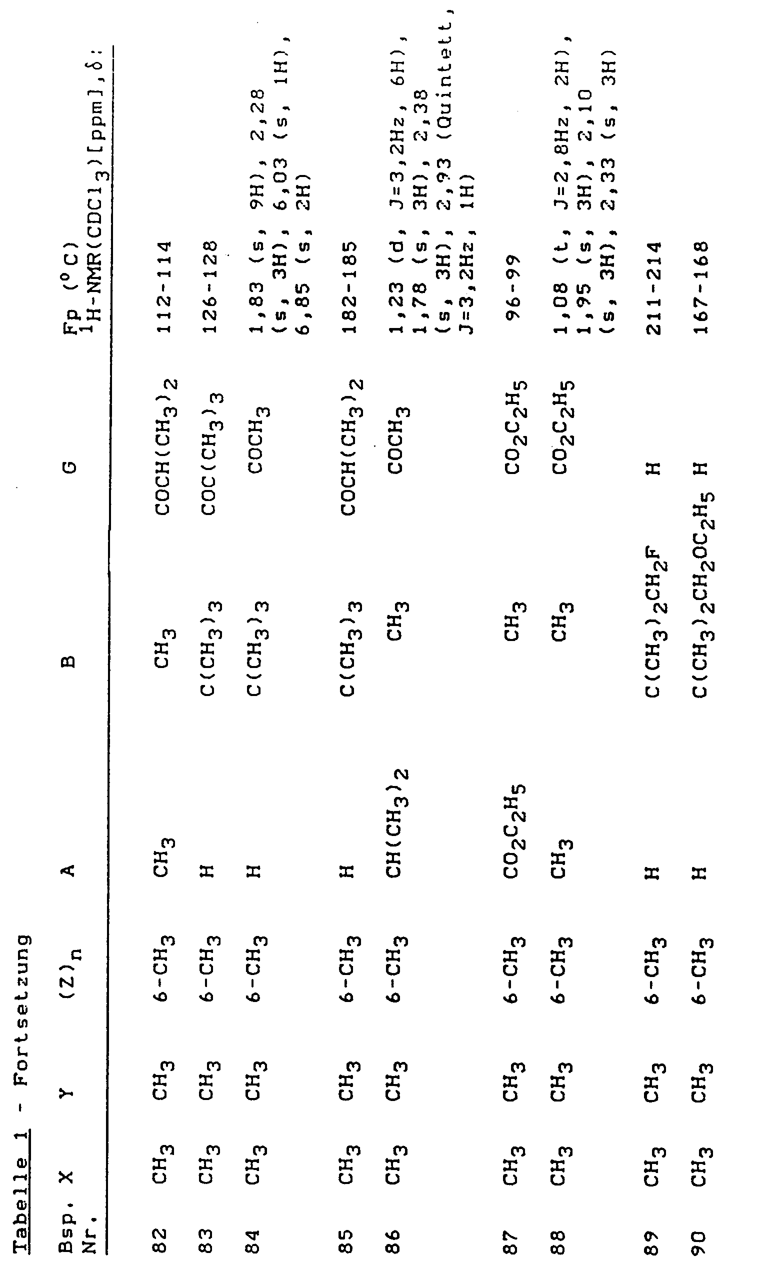

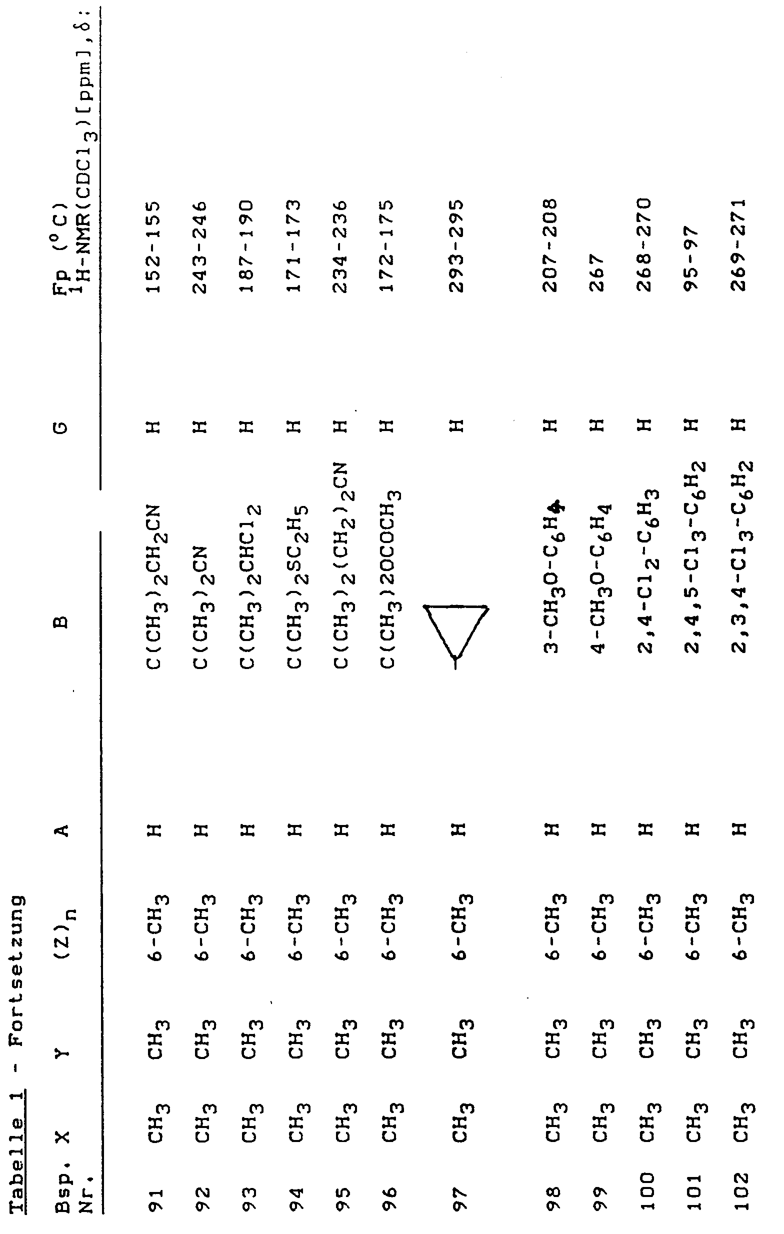

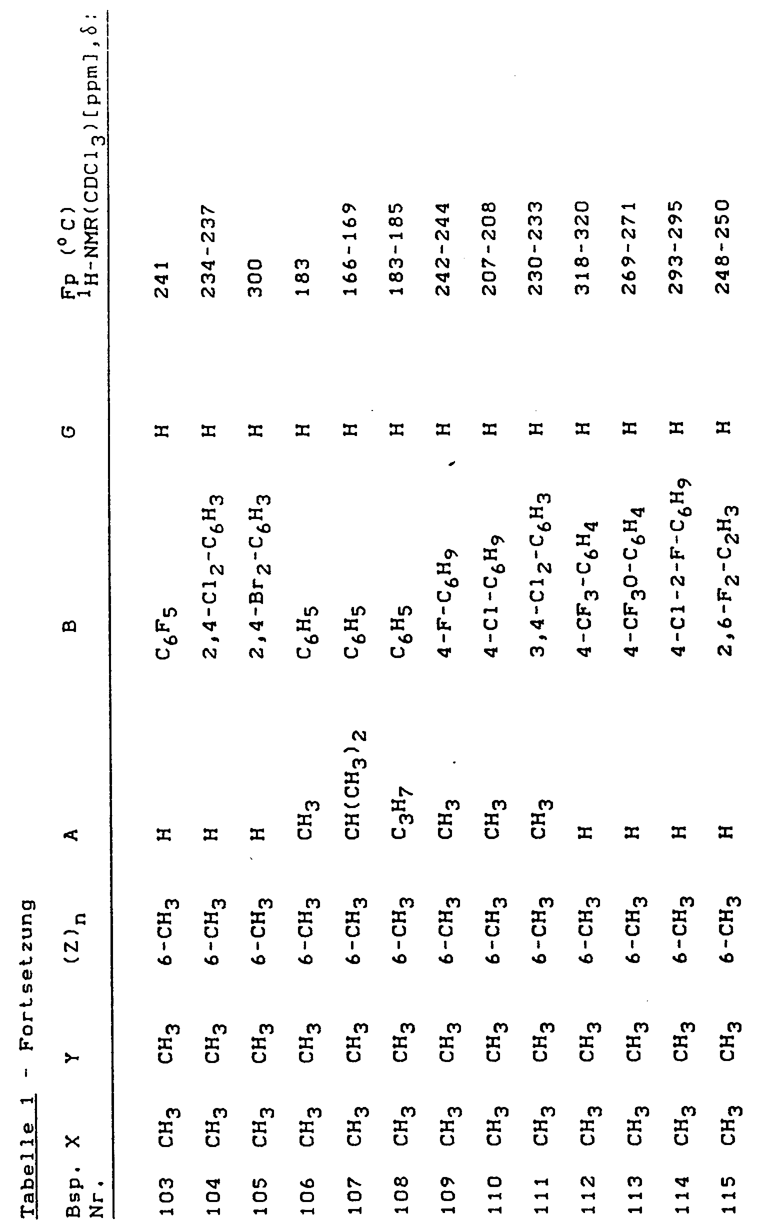

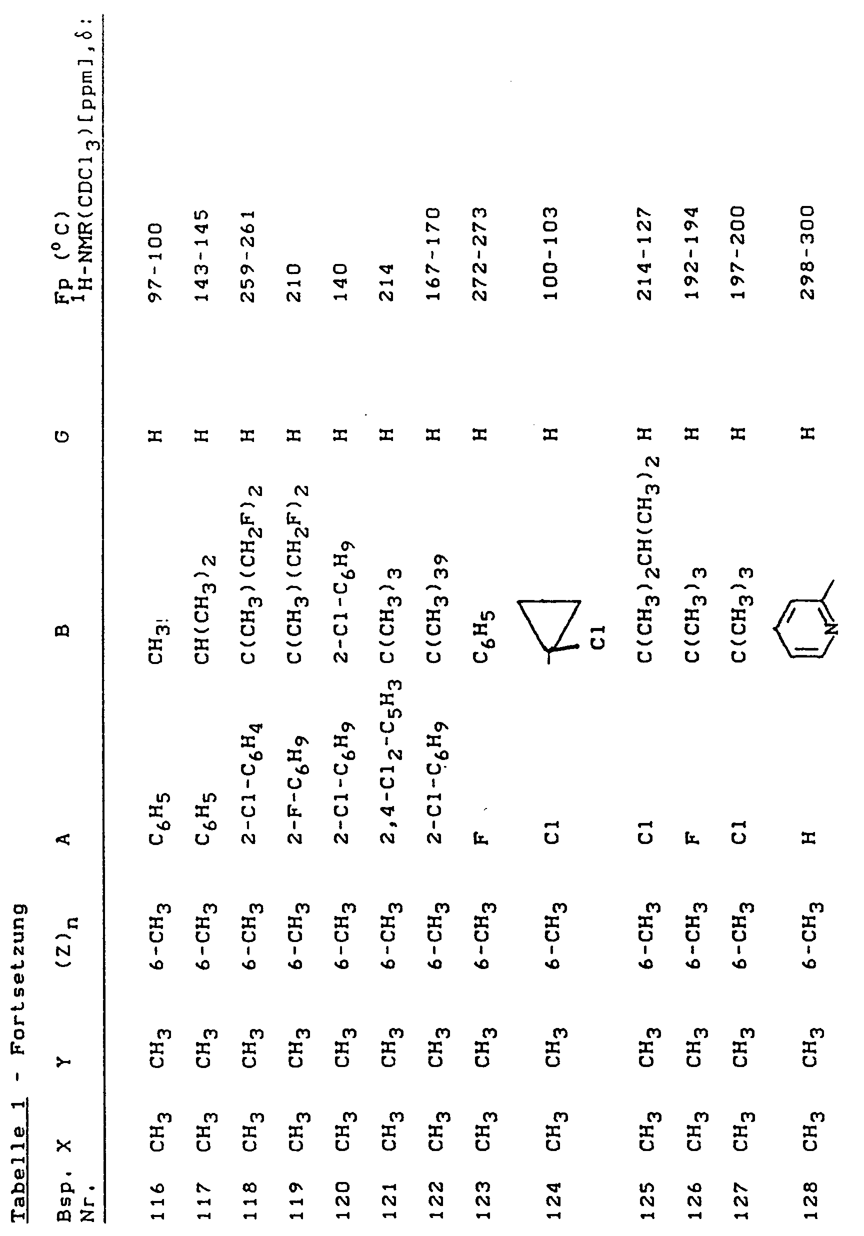

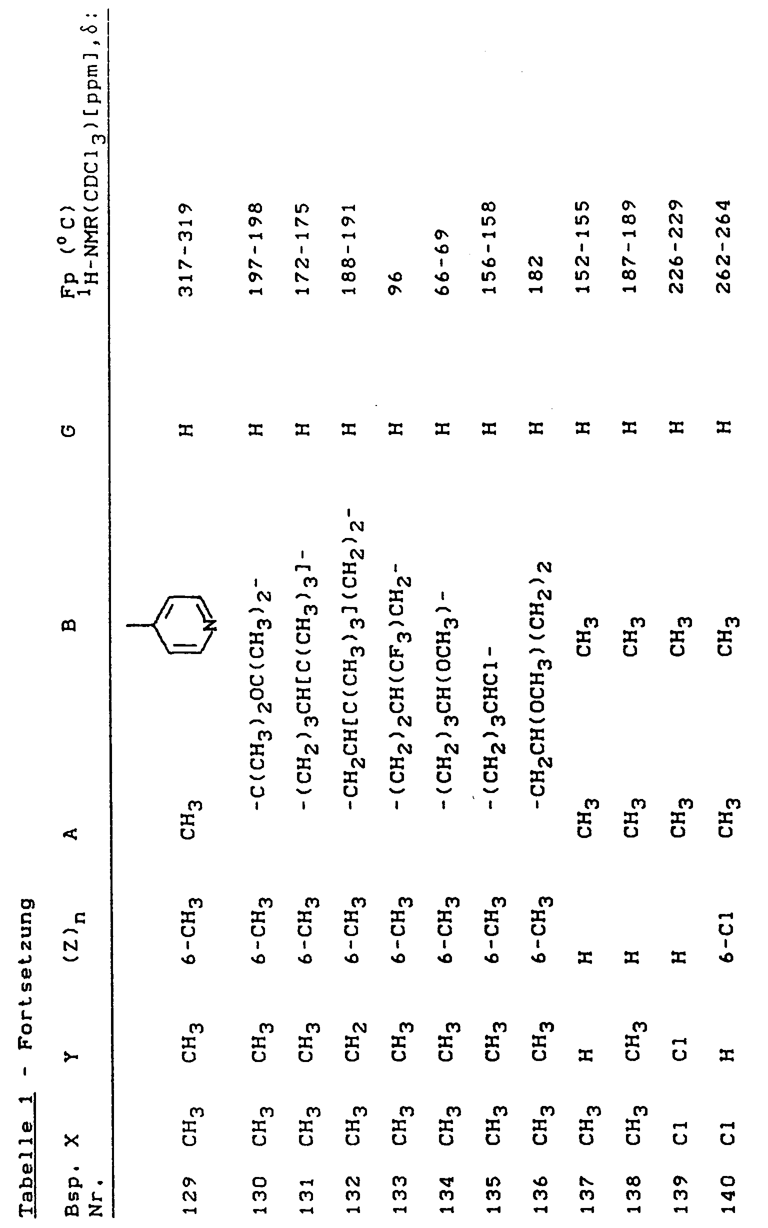

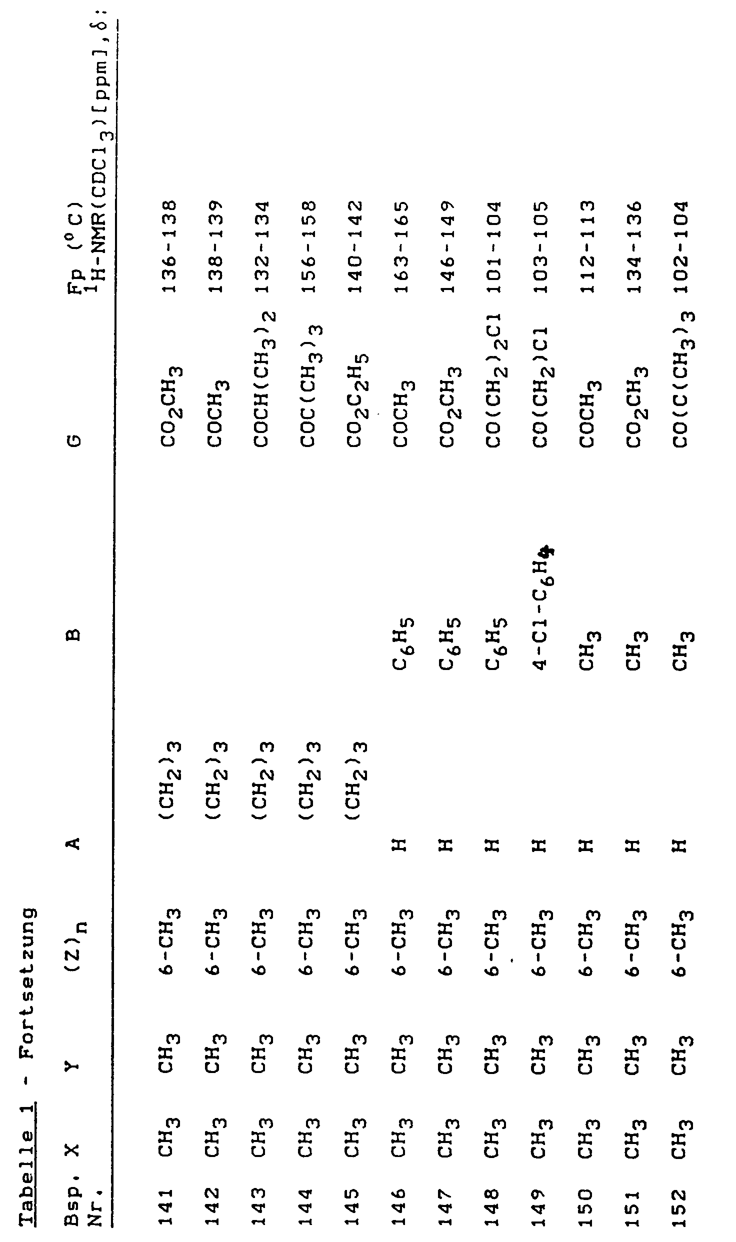

### Herstellung der Ausgangsverbindungen der Formel (III)

### Beispiel (III-1)

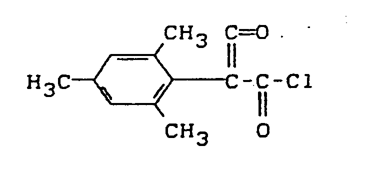

444,5 g (2 Mol) Mesitylmalonsäure werden in 1000 ml Methylcyclohexan bei 75-80°C suspendiert und 714 g (6 Mol) Thionylchlorid innerhalb von 3 Stunden zugetropft. Dann wird langsam weiter erwärmt und 8 Stunden unter Rückflußkühlung bei 110-120°C Badtemperatur nachgerührt.

Überschüssiges Thionylchlorid wird zusammen mit dem Lösungsmittel bei 10 mbar bis 80°C Badtemperatur abdestilliert, der Rückstand nach dem Erkalten mit der 3-fachen Mengen Petrolether verdünnt, filtriert, eingeengt und destilliert.

Man erhält 373 g (84 % der Theorie) Mesitylchlorcarbonylketen vom Siedepunkt 96°/0,45 mbar.

In analoger Weise zu Beispiel (III-1) und unter Berücksichtigung der Angaben in der Beschreibung zu den erfindungsgemäßen Verfahren, können die übrigen Ausgangsverbindungen der Formel (III) hergestellt werden.

### Beispiel A

### Phaedon-Larven-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigten z.B. die Verbindungen der Herstellungsbeispiele 46, 47 und 65 bei einer beispielhaften Konzentration von 0,1 % nach 7 Tagen eine Abtötung von 100 %.

### Beispiel B

### Plutella-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigten z.B. die Verbindungen der Herstellungsbeispiele 38, 46, 52, 53, 61, 81 und 88 bei einer beispielhaften Wirkstoffkonzentration von 0,1 % nach 7 Tagen eine Abtötung von 100 %.

### Beispiel C

### Nephotettix-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigten z.B. die Verbindungen der Herstellungsbeispiele 3, 31, 32, 34, 35, 37, 51, 52, 56, 58, 62, 65, 68, 69, 71, 74, 84 und 85 bei einer beispielhaften Wirkstoffkonzentration von 0,1 % nach 6 Tagen eine Abtötung von 100 %.

### Beispiel D

### Aphis-Test (systemische Wirkung)

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Aphis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigten z.B. die Verbindungen der Herstellungsbeispiele 32 und 38 bei einer beispielhaften Wirkstoffkonzentration von 0,1 % eine Abtötung von 100 %.

### Beispiel E

### Tetranychus-Test (OP-resistent)

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 1OO %, daß alle Spinnmilben abgetötet wurden; O % bedeutet, daß keine Spinnmilben abgetötet wurden.

Bei diesem Test zeigten z.B. die Verbindungen der Herstellungsbeispiele 3, 31, 84, 85 und 88 bei einer beispielhaften Wirkstoffkonzentration von 0,01 % nach 7 Tagen eine Abtötung von 100 %.

### Beispiel F

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
O % = keine Wirkung (wie unbehandelte Kontrolle)
100 % = totale Vernichtung

Bei diesem Test wurden mit einer beispielhaften Aufwandmenge von 500 g/ha bei einer guten bis sehr guten Verträglichkeit durch Soja die folgenden Ergebnisse erhalten:

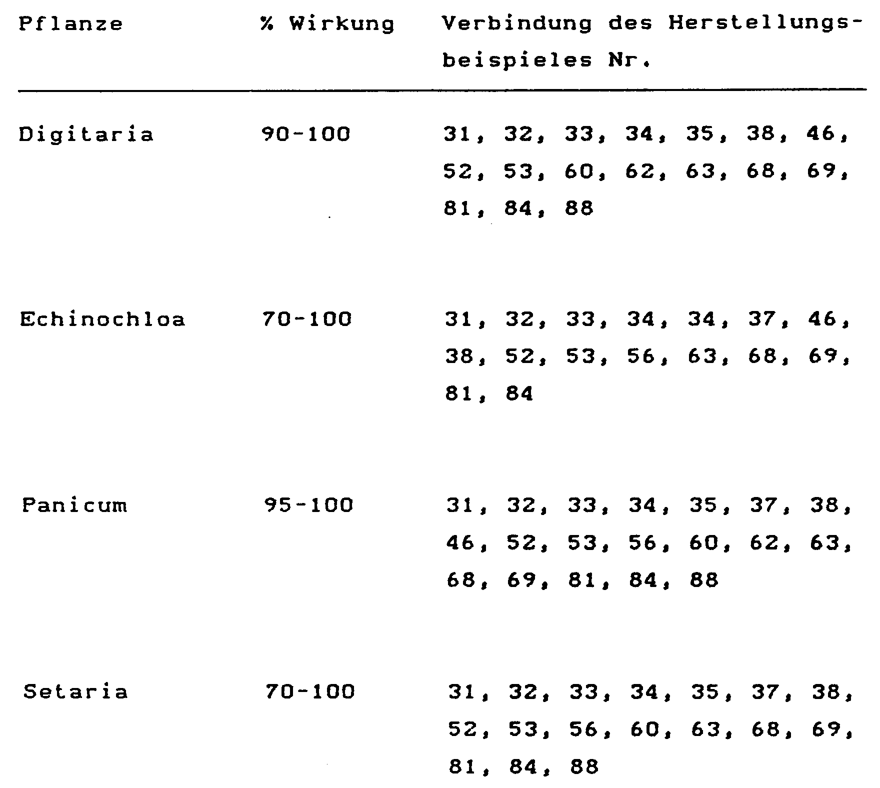

### Beispiel G

### Test mit Lucilia cuprina resistent-Larven

- Emulgator:: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnnt das so erhaltene Konzentrat mit Wasser auf die jeweils gewünschte Konzentration.

Etwa 2O Lucilia cuprina res.-Larven werden in ein Teströhrchen gebracht, welches ca. 1 cm³ Pferdefleisch und O,5 ml der Wirkstoffzubereitung enthält. Nach 24 Stunden wird der Abtötungsgrad bestimmt.

Bei diesem Test zeigten z.B. die Verbindungen der Herstellungsbeispiele 32, 38 und 46 bei einer beispielhaften Wirkstoffkonzentration von 1000 ppm einen Abtötungsgrad von 100 %.

### Beispiel H

### Trichinella spiralis in vitro

Trichinenlarven werden aus der Muskulator von Mäusen isoliert und in 0,9 % NaCl, supplementiert mit 20 µg/ml Sisomycim und 2 µg/ml Clotrimazol, gewaschen. Die eigentliche Inkubation von ca. 20 Trichinen pro Messung erfolgt in 2 ml einer Lösung, bestehend aus 10 g Bacto Casitone, 5 g Hefe (Yeast Extract), 2,5 g Glucose, 0,4 g KH₂PO₄, 0,4 g K₂HPO₄ pro 500 ml pH 7,2, enthaltend 10 µg/ml Sisomycin und 1 mg/ml Clotrimazol. 10 mg der zu testenden Substanz werden in 0,5 ml DMSO gelöst und soviel zum Inkubationsmedium zugesetzt, daß die Endkonzentration 100, 10 und 1 µg/ml beträgt. Nach 5 Tagen Inkubation bei 19°C wird der Versuch ausgewertet. Die Auswertung erfolgt nach folgender Einteilung: 0 = keine Wirkung, die Zahl der lebenden Larven und Würmer ist geringer als bei der Kontrolle; 2 = gute Wirkung, es sind tote Trichinen sichtbar; 3 = sehr gute Wirkung, alle Trichinen sind tot.

Bei diesem Test zeigten z.B. die Verbindungen der Herstellungsbeispiele 13, 14, 15, 18, 26, 37 und 56 bei einer beispielhaften Wirkstoffkonzentration von 100 ppm eine sehr gute Wirkung (100 % Abtötung).

### Beispiel I

### Nippostrongylus in vitro

Nippostrongylus werden aus dem Dünndarm von Ratten isoliert und in 0,9 % NaCl, supplementiert mit 29 µg/ml Sisomycin und 2 µg/ml Clotrimazol gewaschen. Die eigentliche Inkubation von je 5 männlichen und weiblichen Nippostrongylus erfolgt in 1,0 ml Medium, das für die Bestimmung der Aktivität der Acetylcholinesterase genommen wird. Die Enzymbestimmung erfolgt nach der Methode von Rapson et al. 1987 (E.B. Rapson, D.C. Jenkins, A.S. Chilwan), Improved detection of anthelmintic activity in an in vitro screen utilizing adult Nippostrongylus brasiliensis. Parasitol. Res. 1987 73, 190 bis 191.

Für die Bewertung gilt folgendes: 3 = volle Wirkung, 100 % Hemmung des Enzyms; 2 = gute Wirkung, über 75 % Hemmung; 1 = schlechte Wirkung, weniger als 50 % Hemmung.

Bei diesem Test zeigte z.B. die Verbindung des Herstellungsbeispiels 1 bei einer beispielhaften Wirkstoffkonzentration von 100 ppm eine volle Wirkung (100 % Hemmung).

## Patentansprüche

1. 3-Aryl-pyron-Derivate der allgemeinen Formel (I) 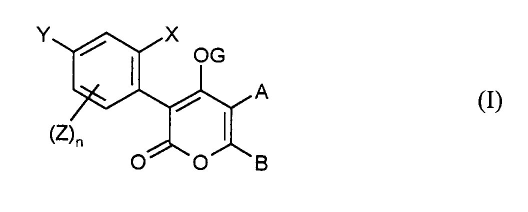 gefunden, in welcher
A für Wasserstoff, Halogen, gegebenenfalls substituierte Reste aus der Reihe Alkyl, Cycloalkyl, Alkenyl, Alkinyl, Arylalkyl, Aryl, Hetarylalkyl und Hetaryl oder für die Gruppen -COR¹, -CO₂R¹, -CN, -CONR¹R², -SO₂R¹ und -P(O)(OR¹)(OR²) steht, worin
R¹ und R² unabhängig voneinander für Wasserstoff oder gegebenenfalls substituierte Reste aus der Reihe Alkyl, Alkenyl, Arylalkyl, Aryl, Hetarylalkyl und Hetaryl stehen oder
R¹ und R² gemeinsam für eine gegebenenfalls substituierte Alkylen-Gruppe stehen, welche durch ein oder mehrere Heteroatome unterbrochen sein kann;
B für Wasserstoff oder gegebenenfalls substituierte Reste aus der Reihe Alkyl, Cycloalkyl, Alkenyl, Alkinyl, Arylalkyl, Aryl, Hetarylalkyl und Hetaryl stehen;
A und B gemeinsam eine gegebenenfalls substituierte Alkylen- oder Alkenylen-Gruppe bilden, welche durch ein oder mehrere Heteroatome oder Heterogruppen unterbrochen sein kann bzw. diese enthalten kann;
X für Halogen, Alkyl oder Alkoxy steht;
Y für Wasserstoff, Halogen, Alkyl, Halogenalkyl oder Alkoxy steht;
Z für Wasserstoff Halogen, Alkyl oder Alkoxy steht,
n für eine ganze Zahl 1, 2 oder 3 steht; und
G für Wasserstoff, ein Metallionenäquivalent, ein Ammonium oder für eine Gruppe -COR³, 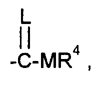 -SO₂-R⁵, 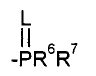 und -CONR⁸R⁹ steht, worin
R³ für gegebenenfalls substituierte Reste aus der Reihe Alkyl, Alkenyl, Alkoxyalkyl, Polyalkoxyalkyl, Alkylthioalkyl gegebenenfalls durch ein oder mehrere Heteroatome unterbrochenes Cycloalkyl, Arylalkyl, Aryl, Hetarylalkyl, Hetaryl, Aryloxyalkyl und Hetaryloxyalkyl steht;
R⁴ für gegebenenfalls substituierte Reste aus der Reihe Alkyl, Alkenyl, Alkoxyalkyl, Polyyalkoxyalkyl, Aryl und Arylalkyl steht;
R⁵, R⁶ und R⁷ unabhängig voneinander für gegebenenfalls substituierte Reste aus der Reihe Alkyl, Alkoxy, Alkylthio, Alkylamino, Dialkylamino, Alkenylthio, Alkinylthio, Cycloalkylthio, Aryl, Aryloxy und Arylthio stehen;
R⁸ und R⁹ unabhängig voneinander für Wasserstoff oder für gegebenenfalls substituierte Reste der Reihe Alkyl, Alkenyl, Cycloalkyl, Alkoxyalkyl, Aryl oder Arylalkyl stehen, oder gemeinsam eine gegebenenfalls substituierte Alkylen-Gruppe bilden, welche durch ein oder mehrere Heteroatome oder Heterogruppen unterbrochen sein kann;
L für Sauerstoff oder Schwefel steht; und
M für Sauerstoff oder Schwefel steht,
ausgenommen die Verbindung der Formel

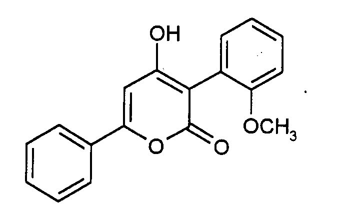

2. Aryl-pyron-Derivate gemäß Anspruch 1, in welchen
A für Wasserstoff; Halogen; gegebenenfalls durch Halogen substituierte Reste aus der Reihe C₁-C₁₀-Alkyl, C₃-C₁₀-Alkenyl, C₃-C₁₀-Alkinyl und C₃-C₁₀-Cycloalkyl, für gegebenenfalls durch Halogen, Nitro, C₁-C₈-Alkyl, C₁-C₈-Alkoxy, C₁-C₈-Alkylthio, C₁-C₈-Halogenalkyl, C₁-C₈-Halogenalkoxy und/oder CN substituierte Reste aus der Reihe Phenyl, Naphthyl, Hetaryl, Phenyl-C₁-C₆-alkyl und Hetaryl-C₁-C₆-alkyl oder für die Gruppen -COR¹, -CO₂R¹, -CN, CONR¹R², -SO₂R¹ und -P(O)(OR¹)(OR²) steht, wobei
R¹ und R² unabhängig voneinander für Wasserstoff, für gegebenenfalls durch Halogen substituierte Reste aus der Reihe C₁-C₁₀-Alkyl und C₃-C₁₀-Alkenyl; für gegebenenfalls durch Halogen, Nitro, C₁-C₈-Alkyl, C₁-C₈-Alkoxy, C₁-C₈-Alkylthio, C₁-C₈-Halogenalkyl, C₁-C₈-Halogenalkoxy und/oder CN substituierte Reste aus der Reihe Phenyl, Naphthyl, Hetaryl, Phenyl-C₁-C₆-alkyl und Hetaryl-C₁-C₆-alkyl stehen; oder R¹ und R² gemeinsam für eine gegebenenfalls durch Stickstoff; Sauerstoff oder Schwefel unterbrochene C₂-C₇-Alkylen-Gruppe stehen;
B für Wasserstoff; für gegebenenfalls durch Halogen, CN, C₁₋₆-Alkoxy, C₁₋₆-Alkylthio, C₁-C₆-Alkylcarbonyloxy oder Phenyl substituierte Reste aus der Reihe C₁-C₁₀-Alkyl, C₃-C₁₀-Alkenyl, C₃-C₁₀-Alkinyl und C₃-C₁₀-Cycloalkyl oder für gegebenenfalls durch Halogen, CN, Nitro, C₁-C₈-Alkyl, C₁-C₈-Alkoxy, C₁-C₈-Alkylthio, C₁-C₈-Halogenalkyl und/oder C₁-C₈-Halogenalkoxy substituierte Reste aus der Reihe Phenyl, Naphthyl, Hetaryl, Phenyl-C₁-C₆-alkyl und Hetaryl-C₁-C₆-alkyl steht, und
A und B gemeinsam eine gegebenenfalls durch C₁-C₈-Alkyl, C₁₋₆-Halogenalkyl, C₁₋₆-Alkoxy, C₁₋₆-Alkylthio und/oder Halogen substituierte geradkettige oder verzweigte C₂-C₇-Alkylen- oder C₂-C₇-Alkenylen-Gruppe bilden, welche durch Stickstoff; Schwefel, Sauerstoff oder -O-CO- unterbrochen sein kann bzw. diese enthalten kann;
X für Halogen, C₁-C₆-Alkyl oder C₁-C₆-Alkoxy steht;
Y für Wasserstoff Halogen, C₁-C₆-Alkyl, C₁-C₆-Alkoxy oder C₁-C₆-Halogenalkyl steht;
Z für Wasserstoff, Halogen, C₁-C₆-Alkyl oder C₁-C₆-Alkoxy steht;
n für 1 oder 2 steht;
G für Wasserstoff, -COR³, 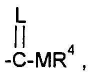 -SO₂-R⁵, 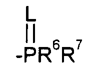 und -CONR⁸R⁹ steht,
worin
R³ für gegebenenfalls durch Halogen substituierte Reste aus der Reihe C₁-C₂₀-Alkyl, C₂-C₂₀-Alkenyl, C₁-C₈-Alkoxy-C₁-C₈-alkyl, C₁-C₈-Alkylthio-C₁-C₈-alkyl, C₁-C₈-Polyalkoxy-C₁-C₈-alkyl und gegebenenfalls durch Sauerstoff und/oder Schwefel unterbrochenes C₃-C₈-Cycloalkyl; für gegebenenfalls durch Halogen, Nitro, C₁-C₆-Alkyl, C₁-C₆-Alkoxy, C₁-C₆-Halogenalkyl und/oder C₁-C₆-Halogenalkoxy substituiertes Phenyl; für gegebenenfalls durch Halogen, C₁-C₆-Alkyl, C₁-C₆-Alkoxy, C₁-C₆-Halogenalkyl und/oder C₁-C₆-Halogenalkoxy substituiertes Phenyl-C₁-C₆-alkyl; für gegebenenfalls durch Halogen und/oder C₁-C₆-Alkyl substituiertes Hetaryl; für gegebenenfalls durch Halogen und/oder C₁-C₆-Alkyl substituiertes Phenoxy-C₁-C₆-alkyl oder für gegebenenfalls durch Halogen, Amino und/oder C₁-C₆-Alkyl substituiertes Hetaryloxy-C₁-C₆-alkyl steht;
R⁴ für gegebenenfalls durch Halogen substituierte Reste aus der Reihe C₁-C₂₀-Alkyl, C₂-C₂₀-Alkenyl, C₁-C₈-Alkoxy-C₁-C₈-alkyl, C₁-C₈-Polyalkoxy-C₁-C₈-alkyl oder für gegebenenfalls durch Halogen, Nitro, C₁-C₆-Alkyl, C₁-C₆-Alkoxy und/oder C₁-C₆-Halogenalkyl substituierte Reste aus der Reihe Phenyl und Benzyl steht;
R⁵, R⁶ und R⁷ unabhängig voneinander für gegebenenfalls durch Halogen substituierte Reste aus der Reihe C₁-C₈-Alkyl, C₁-C₈-Alkoxy, C₁-C₈-Alkylthio, C₁-C₈-Alkylamino, Di-(C₁-C₈)-alkylamino, C₂-C₅-Alkenylthio, C₂-C₅-Alkinylthio, C₃-C₇-Cycloalkylthio oder für gegebenenfalls durch Halogen, Nitro, Cyano, C₁-C₄-Alkoxy, C₁-C₄-Alkylthio, C₁-C₄-Halogenalkoxy, C₁-C₄-Halogenalkylthio, C₁-C₄-Alkyl und/oder C₁-C₄-Halogenalkyl substituierte Reste aus der Reihe Phenyl, Phenyloxy und Phenylthio stehen;
R⁸ und R⁹ unabhängig voneinander für Wasserstoff für gegebenenfalls durch Halogen substituierte Reste aus der Reihe C₁-C₂₀-Alkyl, C₁-C₂₀-Alkoxy, C₃-C₁₀-Cycloalkyl, C₂-C₈-Alkenyl, C₁-C₈-Alkoxy-C₁-C₈-alkyl oder für gegebenenfalls durch Halogen, C₁-C₂₀-Alkyl, C₁-C₂₀-Halogenalkyl und/oder C₁-C₂₀-Alkoxy substituierte Reste aus der Reihe Phenyl und Benzyl stehen oder R⁸ und R⁹ gemeinsam eine C₂-C₆-Alkylengruppe bilden, welche durch Sauerstoff und/oder Schwefel unterbrochen sein kann;
L für Sauerstoff oder Schwefel steht, und
M für Sauerstoff oder Schwefel steht,
ausgenommen die Verbindung der Formel

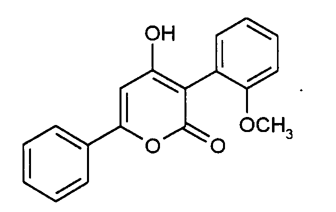

3. Aryl-pyron-Derivate gemäß Anspruch 1, in welchen
A für Wasserstoff; Halogen; gegebenenfalls durch Halogen substituierte Reste aus der Reihe C₁-C₈-Alkyl, C₃-C₈-Alkenyl, C₃-C₈-Alkinyl und C₃-C₈-Cycloalkyl; für gegebenenfalls durch Halogen, Nitro, C₁-C₄-Alkyl, C₁-C₄-Alkoxy, C₁-C₄-Alkylthio, C₁-C₄-Halogenalkyl, C₁-C₄-Halogenalkoxy und/oder CN substituierte Reste aus der Reihe Phenyl, Naphthyl, Hetaryl, Phenyl-C₁-C₄-alkyl und Hetaryl-C₁-C₄-alkyl oder für die Gruppen -COR¹, -CO₂R¹, -CN, -CONR¹R², -SO₂R¹ und -P(O)(OR¹)(OR²) steht, wobei
R¹ und R² unabhängig voneinander für Wasserstoff, für gegebenenfalls durch Halogen substituierte Reste aus der Reihe C₁-C₈-Alkyl und C₃-C₈-Alkenyl; für gegebenenfalls durch Halogen, Nitro, C₁-C₄-Alkyl, C₁-C₄-Alkoxy, C₁-C₄-Alkylthio, C₁-C₄-Halogenalkyl, C₁-C₄-Halogenalkoxy und/oder CN substituierte Reste aus der Reihe Phenyl, Hetaryl, Phenyl-C₁-C₄-alkyl und Hetaryl-C₁-C₄-alkyl stehen; oder R¹ und R² gemeinsam für eine gegebenenfalls durch Stickstoff; Sauerstoff oder Schwefel unterbrochene C₂-C₆-Alkylengruppe stehen;
B für Wasserstoff; für gegebenenfalls durch Halogen, C₁₋₄-Alkoxy, C₁₋₄-Alkylthio, C₁-C₄-Alkylcarbonyloxy oder Phenyl substituierte Reste aus der Reihe C₁-C₈-Alkyl, C₃-C₈-Alkenyl, C₃-C₈-Alkinyl und C₃-C₈-Cycloalkyl oder für gegebenenfalls durch Halogen, CN, Nitro, C₁-C₆-Alkyl, C₁-C₆-Alkoxy, C₁-C₆-Alkylthio, C₁-C₆-Halogenalkyl und/oder C₁-C₆-Halogenalkoxy substituierte Reste aus der Reihe Phenyl, Naphthyl, Hetaryl, Phenyl-C₁-C₄-alkyl und Hetaryl-C₁-C₄-alkyl steht; und
A und B gemeinsam eine gegebenenfalls durch 1 bis 5 Halogen-C₁-C₄-alkyl, C₁₋₄-Alkoxy, C₁-C₄-Alkyl und/oder Halogen substituierte C₂-C₆-Alkylen- oder C₂-C₆-Alkenylen-Gruppe bilden, welche durch Stickstoff; Schwefel, Sauerstoff oder -O-CO- unterbrochen sein kann bzw. diese enthalten kann,
X für Halogen, C₁-C₄-Alkyl oder C₁-C₄-Alkoxy steht;
Y für Wasserstoff; Halogen, C₁-C₄-Alkyl, C₁-C₄-Alkoxy oder C₁-C₄-Halogenalkyl steht;
Z für Wasserstoff; Halogen, C₁-C₄-Alkyl oder C₁-C₄-Alkoxy steht;
n für 1 oder 2 steht;
G für Wasserstoff; -COR³, 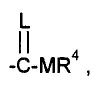 -SO₂-R⁵, 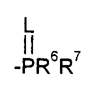 und -CONR⁸R⁹ steht,
worin
R³ für gegebenenfalls durch Halogen substituierte Reste aus der Reihe C₁-C₁₆-Alkyl, C₂-C₁₆-Alkenyl, C₁-C₆-Alkoxy-C₁-C₆-alkyl, C₁-C₆-Alkylthio-C₁-C₆-alkyl, C₁-C₆-Polyalkoxy-C₁-C₆-alkyl und gegebenenfalls durch Sauerstoff und/oder Schwefel unterbrochenes C₃-C₇-Cycloalkyl; für gegebenenfalls durch Halogen, Nitro, C₁-C₄-Alkyl, C₁-C₄-Alkoxy, C₁-C₃-Halogen-alkyl und/oder CF₁-C₃-Halogenalkoxyy substituiertes Phenyl; für gegebenenfalls durch Halogen, C₁-C₄-Alkyl, C₁-C₄-Alkoxy, C₁-C₃-Halogenalkyl und/oder C₁-C₃-Halogenalkoxy substituiertes Phenyl-C₁-C₄-alkyl; für gegebenenfalls durch Halogen und/oder C₁-C₆-Alkyl substituiertes Hetaryl; für gegebenenfalls durch Halogen und/oder C₁-C₄-Alkyl substituiertes Phenoxy-C₁-C₅-alkyl oder für gegebenenfalls durch Halogen, Amino und/oder C₁-C₄-Alkyl substituiertes Hetaryloxy-C₁-C₅-alkyl steht;
R⁴ für gegebenenfallsdurch Halogen substituierte Reste aus der Reihe C₁-C₁₆-Alkyl, C₂-C₁₆-Alkenyl, C₁-C₁₆-Alkoxy-C₁-C₆-alkyl, C₁-C₆-Polyalkoxy-C₁-C₆-alkyl oder für gegebenenfalls durch Halogen, Nitro, C₁-C₄-Alkyl, C₁-C₃-Alkoxy und/oder C₁-C₃-Halogenalkyl substituierte Reste aus der Reihe Phenyl und Benzyl steht;
R⁸ und R⁹ unabhängig voneinander für Wasserstoff, für gegebenenfalls durch Halogen substituierte Reste aus der Reihe C₁-C₁₆-Alkyl, C₁-C₁₆-Alkoxy, C₃-C₁₆-Cycloalkyl, C₂-C₆-Alkenyl, C₁-C₆-Alkoxy-C₁-C₆-alkyl oder für gegebenenfalls durch Halogen, C₁-C₆-Alkyl, C₁-C₆-Halogenalkyl und/oder C₁-C₆-Alkoxy substituierte Reste aus der Reihe Phenyl und Benzyl stehen, oder R⁸ und R⁹ gemeinsam eine C₂-C₆-Alkylen-Gruppe bilden, welche durch Sauerstoff und/oder Schwefel unterbrochen sein kann;
L für Sauerstoff oder Schwefel steht; und
M für Sauerstoff oder Schwefel steht,
ausgenommen die Verbindung der Formel

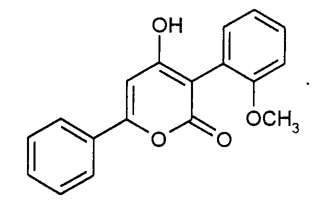

4. 3-Aryl-pyron-Derivate gemäß Anspruch 1, in welchen
A für Wasserstoff, Halogen, gegebenenfalls durch Halogen substituierte Reste aus der Reihe C₁-C₄-Alkyl, Phenyl und Benzyl steht oder A und B gemeinsam eine gegebenenfalls durch Sauerstoff oder Schwefel unterbrochene C₃-C₅-Alkenylgruppe bilden;
B für Wasserstoff, für gegebenenfalls durch Halogen, CN, Methoxy, Methylthio substituiertes C₁-C₄-Alkyl oder für gegebenenfalls durch Halogen, Nitro, C₁-C₄-Alkyl und/oder C₁-C₄-Alkoxy substituierte Reste aus der Reihe Phenyl, Furyl, Thiazolyl und Pyridyl steht; und A und B gemeinsam die Gruppen -(CH₂)₃-, -(CH₂)₄-, -(CH₂)₅-, -CH₂-CH(CH₃)(CH₂)₂-, -CH₂O-(CH₂)₂- oder CH₂S(CH₂)₂-, die durch CF₃, Cl oder Methoxy substituiert sein können, bilden;
X für Methyl steht;
Y für Methyl steht;
Z für Methyl steht; und
G für Wasserstoff oder COR³ steht, worin
R³ für C₁-C₆-Alkyl oder C₁-C₆-Alkoxy steht.

5. Verfahren zur Herstellung der 3-Aryl-pyron-Derivate gemäß Anspruch 1, dadurch gekennzeichnet, daß man
a) zur Herstellung der Verbindungen, in welchen G für Wasserstoff steht,
Carbonylverbindungen der allgemeinen Formel (II) 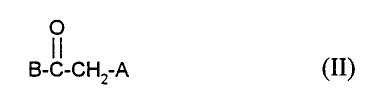 in welcher
A und B die oben angegebene Bedeutung haben,
mit Ketensäurehalogeniden der allgemeinen Formel (III) 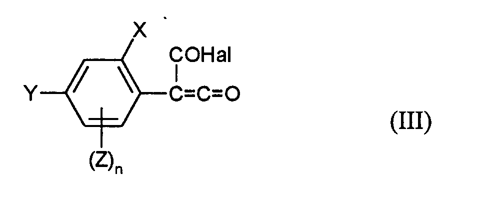 in welcher
X, Y, Z und n die oben angegebenen Bedeutungen haben und
Hal für Halogen steht
in Gegenwart eines Verdünnungsmittels und gegebenenfalls in Gegenwart eines Säureakzeptors umsetzt; und
b) zur Herstellung der Verbindungen der allgemeinen Formel (I), in welcher G für -COR³ steht,
Verbindungen der allgemeinen Formel (I), in welchen G für Wasserstoff steht (erhältlich nach Variante a))
α) mit Säurehalogeniden der allgemeinen Formel (IV) 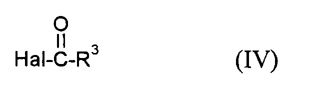 in welcher
R³ die oben angegebene Bedeutung hat und
Hal für Halogen steht, oder
β) mit Carbonsäureanhydriden der allgemeinen Formel (V) 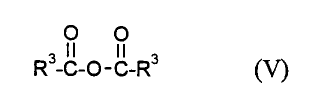 in welcher
R³ die oben angegebene Bedeutung hat,
gegebenenfalls in Gegenwart eines Verdünnungsmittels und gegebenenfalls in Gegenwart eines Säurebindemittels umsetzt; und
c) zur Herstellung der Verbindungen der allgemeinen Formel (I), in welcher G für -C(L)-MR⁴ steht, worin L Sauerstoff bedeutet,
Verbindungen der allgemeinen Formel (I), in welchen G für Wasserstoff steht (erhältlich nach Variante a)),
mit Verbindungen der allgemeinen Formel (VI) 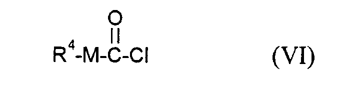 in welcher
R⁴ und M die oben angegebenen Bedeutungen haben,
gegebenenfalls in Gegenwart eines Verdünnungsmittels und gegebenenfalls in Gegenwart eines Säurebindemittels umsetzt; und
d) zur Herstellung der Verbindungen der allgemeinen Formel (I), in welchen G für -C(L)-MR⁴ steht, worin L Schwefel bedeutet,
Verbindungen der allgemeinen Formel (I), in welchen G für Wasserstoff steht (erhältlich nach Variante a)),
α) mit Verbindungen der allgemeinen Formel (VII) 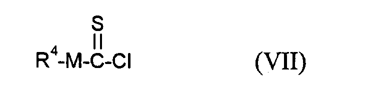 in welcher
R⁴ und M die oben angegebenen Bedeutungen haben,
gegebenenfalls in Gegenwart eines Verdünnungsmittels und gegebenenfalls in Gegenwart eines Säurebindemittels umsetzt, oder
β) mit Schwefelkohlenstoff (CS₂) und anschließend mit Alkylhalogeniden der allgemeinen Formel (VIII)
R⁴-Hal¹ (VIII)
in welcher
R⁴ die oben angegebene Bedeutung hat und
Hal¹ für Halogen steht,
gegebenenfalls in Gegenwart eines Verdünnungsmittels und gegebenenfalls in Gegenwart eines Säurebindemittels umsetzt; und
e) zur Herstellung von Verbindungen der allgemeinen Formel (I), in welcher G für -SO₂R⁵ steht,
Verbindungen der allgemeinen Formel (I), in welchen G für Wasserstoff steht (erhältlich nach Variante a)),
mit Sulfonsäurechloriden der allgemeinen Formel (IX)
R⁵-SO₂-Cl (IX)
in welcher
R⁵ die oben angegebene Bedeutung hat,
gegebenenfalls in Gegenwart eines Verdünnungsmittels und gegebenenfalls in Gegenwart eines Säurebindemittels umsetzt; und
f) zur Herstellung von Verbindungen der allgemeinen Formel (I), in welchen G für -P(L)R⁶R⁷ steht,
Verbindungen der allgemeinen Formel (I), in welchen G für Wasserstoff steht (erhältlich nach Variante a)),
mit Verbindungen der allgemeinen Formel (X) 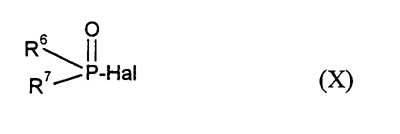 in welcher
R⁶, R⁷ und L die oben angegebenen Bedeutungen haben und
Hal für Halogen steht,
gegebenenfalls in Gegenwart eines Verdünnungsmittels und gegebenenfalls in Gegenwart eines Säurebindemittels umsetzt; und
g) zur Herstellung von Verbindungen der allgemeinen Formel (I), in welchen G für ein Metallionenäquivalent oder für ein Ammoniumion steht,
Verbindungen der allgemeinen Formel (I), in welchen G für Wasserstoff steht (erhältlich nach Variante a)),
mit
Metallhydroxiden oder Aminen umsetzt; und
h) zur Herstellung von Verbindungen der allgemeinen Formel (I), in welchen G für -C(L)NR⁸R⁹ steht,
Verbindungen der allgemeinen Formel (I), in welchen G fuhr Wasserstoff steht (erhältlich nach Variante a)),
α) mit Verbindungen der allgemeinen Formel (XI)
R⁸-N=C=L (XI)
in welcher
R⁸ und L die oben angegebenen Bedeutungen haben,
gegebenenfalls in Gegenwart eines Verdünnungsmittels und gegebenenfalls in Gegenwart eines Katalysators umsetzt; oder
β) mit Verbindungen der allgemeinen Formel (XII) 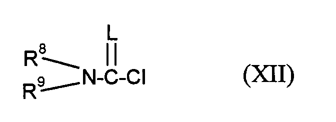 in welcher
R⁸, R⁹ und L die oben angegebenen Bedeutungen haben,
gegebenenfalls in Gegenwart eines Verdünnungsmittels und gegebenenfalls in Gegenwart eines Säurebindemittels umsetzt.

6. Schädlingsbekämpffingsmittel, gekennzeichnet durch einen Gehalt an mindestens einer Verbindung der Formel (I) gemäß Anspruch 1.

7. Arthropodizide, nematizide und herbizide Mittel, gekennzeichnet durch einen Gehalt an mindestens einer Verbindung der Formel (I).

8. Verwendung von Verbindungen der Formel (I) gemäß Anspruch 1 zur Bekämpfung von Schädlingen im Pflanzenschutz, Haushaltsbereich, Hygienebereich und Vorratsschutz.

9. Verwendung von Verbindungen der Formel (I) gemäß Anspruch 1 zur Bekämpfung von Arthropoden, Nematoden und unerwünschtem Pflanzenbewuchs im Pflanzenschutz, Haushaltsbereich, Hygienebereich und Vorratsschutz.

10. Verfahren zur Bekämpfung von Schädlingen im Pflanzenschutz, Haushaltsbereich, Hygienebereich und Vorratsschutz, dadurch gekennzeichnet, daß man Verbindungen der Formel (I) gemäß Anspruch 1 auf die Schädlinge und/oder ihren Lebensraum einwirken läßt.

11. Verfahren zur Herstellung von Schädlingsbekämpfungsmitteln, dadurch gekennzeichnet, daß man Verbindungen der Formel (I) gemäß Anspruch 1 mit Streckmitteln und/oder oberflächenaktiven Mitteln vermischt.

12. Verwendung von Verbindungen der Formel (I) gemäß Anspruch 1 zur Herstellung von Schädlingsbekämfungsmitteln zur Bekämpfung von Ekto- und Endoparasiten.

## Claims

1. 3-Aryl-pyrone derivatives of the general formula (I) 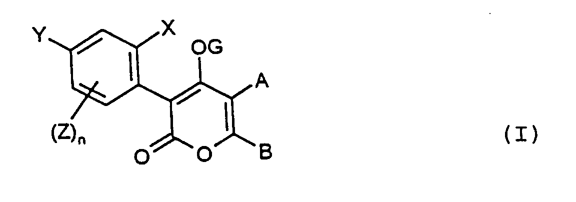 in which
A represents hydrogen, halogen, optionally substituted radicals from the series comprising alkyl, cycloalkyl, alkenyl, alkinyl, arylalkyl, aryl, hetarylalkyl and hetaryl, or represents the groups -COR¹, -CO₂R¹, -CN, -CONR¹R², -SO₂R¹ and -P(O)(OR¹)(OR²), in which
R¹ and R² independently of one another represent hydrogen or optionally substituted radicals from the series comprising alkyl, alkenyl, arylalkyl, aryl, hetarylalkyl and hetaryl or
R¹ and R² together represent an optionally substituted alkylene group which can be interrupted by one or more hetero atoms;
B represents hydrogen or optionally substituted radicals from the series comprising alkyl, cycloalkyl, alkenyl, alkinyl, arylalkyl, aryl, hetarylalkyl and hetaryl;
A and B together form an optionally substituted alkylene or alkenylene group, each of which can be interrupted by, or contain, one or more hetero atoms or hetero groups;
X represents halogen, alkyl or alkoxy;
Y represents hydrogen, halogen, alkyl, halogenoalkyl or alkoxy;
Z represents hydrogen, halogen, alkyl or alkoxy;
n represents an integer 1, 2 or 3; and
G represents hydrogen, a metal ion equivalent, an ammonium ion or a group -COR³, 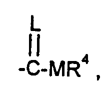 -SO₂-R⁵, 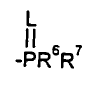 and -CONR⁸R⁹,
in which
R³ represents optionally substituted radicals from the series comprising alkyl, alkenyl, alkoxyalkyl, polyalkoxyalkyl, alkylthioalkyl, cycloalkyl which is optionally interrupted by one or more hetero atoms, arylalkyl, aryl, hetarylalkyl, hetaryl, aryloxyalkyl and
R⁴ represents optionally substituted radicals from the series comprising alkyl, alkenyl, alkoxyalkyl, polyalkoxyalkyl, aryl and arylalkyl;
R⁵, R⁶ and R⁷ independently of one another represent optionally substituted radicals from the series comprising alkyl, alkoxy, alkylthio, alkylamino, dialkylamino, alkenylthio, alkinylthio, cycloalkylthio, aryl, aryloxy and arylthio;
R⁸ and R⁹ independently of one another represent hydrogen or optionally substituted radicals from the series comprising alkyl, alkenyl, cycloalkyl, alkoxyalkyl, aryl or arylalkyl, or together form an optionally substituted alkylene group which can be interrupted by one or more hetero atoms or hetero groups;
L represents oxygen or sulphur; and
M represents oxygen or sulphur,
with the exception of the compound of the formula

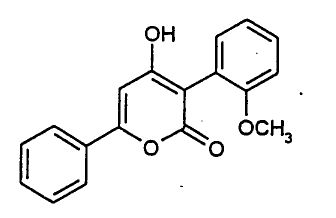

2. Aryl-pyrone derivatives according to Claim 1, in which
A represents hydrogen; halogen; radicals from the series comprising C₁-C₁₀-alkyl, C₃-C₁₀-alkenyl, C₃-C₁₀-alkinyl and C₃-C₁₀-cycloalkyl, each of which is optionally substituted by halogen; radicals from the series comprising phenyl, naphthyl, hetaryl, phenyl-C₁-C₆-alkyl and hetaryl-C₁-C₆-alkyl, each of which is optionally substituted by halogen, nitro, C₁-C₈-alkyl, C₁-C₈-alkoxy, C₁-C₈-alkylthio, C₁-C₈-halogenoalkyl, C₁-C₈-halogenoalkoxy and/or CN, or represents the groups -COR¹, -CO₂R¹, -CN, -CONR¹R², -SO₂R¹ and -P(O)(OR¹)(OR²), in which
R¹ and R² independently of one another represent hydrogen, radicals from the series comprising C₁-C₁₀-alkyl and C₃-C₁₀-alkenyl, each of which is optionally substituted by halogen; or represent radicals from the series comprising phenyl, naphthyl, hetaryl, phenyl-C₁-C₆-alkyl and hetaryl-C₁-C₆-alkyl, each of which is optionally substituted by halogen, nitro, C₁-C₈-alkyl, C₁-C₈-alkoxy, C₁-C₈-alkylthio, C₁-C₈-halogenoalkyl, C₁-C₈-halogenoalkoxy and/or CN; or R¹ and R² together represent a C₂-C₇-alkylene group which is optionally interrupted by nitrogen, oxygen or sulphur;
B represents hydrogen; radicals from the series comprising C₁-C₁₀-alkyl, C₃-C₁₀-alkenyl, C₃-C₁₀-alkinyl and C₃-C₁₀-cycloalkyl which are optionally substituted by halogen, CN, C₁₋₆-alkoxy, C₁₋₆-alkylthio, C₁-C₆-alkylcarbonyloxy or phenyl, or represents radicals from the series comprising phenyl, naphthyl, hetaryl, phenyl-C₁-C₆-alkyl and hetaryl-C₁-C₆-alkyl which are optionally substituted by halogen, CN, nitro, C₁-C₈-alkyl, C₁-C₈-alkoxy, C₁-C₈-alkylthio, C₁-C₈-halogenoalkyl and/or C₁-C₈-halogenoalkoxy, and
A and B together represent a straight-chain or branched C₂-C₇-alkylene or C₂-C₇-alkenylene group which is optionally substituted by C₁-C₈-alkyl, C₁₋₆-halogenolakyl, C₁₋₆-alkoxy, C₁₋₆-alkylthio and/or halogen and which can optionally be interrupted by, or contain, nitrogen, sulphur, oxygen or -O-CO-;
X represents halogen, C₁-C₆-alkyl or C₁-C₆-alkoxy;
Y represents hydrogen, halogen, C₁-C₆-alkyl, C₁-C₆-alkoxy or C₁-C₆-halogenoalkyl;
Z represents hydrogen, halogen, C₁-C₆-alkyl or C₁-C₆-alkoxy;
n represents 1 or 2;
G represents hydrogen or -COR³, 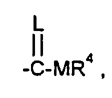 -SO₂-R⁵, 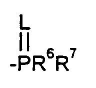 and -CONR⁸R⁹
in which
R³ represents radicals from the series comprising C₁-C₂₀-alkyl, C₂-C₂₀-alkenyl, C₁-C₈-alkoxy-C₁-C₈-alkyl, C₁-C₈-alkylthio-C₁-C₈-alkyl, C₁-C₈-polyalkoxy-C₁-C₈-alkyl and C₃-C₈-cycloalkyl which is optionally interrupted by oxygen and/or sulphur, each of these radicals optionally being substituted by halogen; phenyl which is optionally substituted by halogen, nitro, C₁-C₆-alkyl, C₁-C₆-alkoxy, C₁-C₆-halogenoalkyl and/or C₁-C₆-halogenoalkoxy; phenyl-C₁-C₆-alkyl which is optionally substituted by halogen, C₁-C₆-alkyl, C₁-C₆-alkoxy, C₁-C₆-halogenoalkyl and/or C₁-C₆-halogenoalkoxy; hetaryl which is optionally substituted by halogen and/or C₁-C₆-alkyl; phenoxy-C₁-C₆-alkyl which is optionally substituted by halogen and/or C₁-C₆-alkyl, or hetaryloxy-C₁-C₆-alkyl which is optionally substituted by halogen, amino and/or C₁-C₆-alkyl;
R⁴ represents radicals from the series comprising C₁-C₂₀-alkyl, C₂-C₂₀-alkenyl, C₁-C₈-alkoxy-C₁-C₈-alkyl or C₁-C₈-polyalkoxy-C₁-C₈-alkyl which are optionally substituted by halogen, or radicals from the series comprising phenyl and benzyl which are optionally substituted by halogen, nitro, C₁-C₆-alkyl, C₁-C₆-alkoxy and/or C₁-C₆-halogenoalkyl;
R⁵, R⁶ and R⁷ independently of one another represent radicals from the series comprising C₁-C₈-alkyl, C₁-C₈-alkoxy, C₁-C₈-alkylthio, C₁-C₈-alkylamino, di-(C₁-C₈)-alkylamino, C₂-C₅-alkenylthio, C₂-C₅-alkinylthio or C₃-C₇-cycloalkylthio which are optionally substituted by halogen, or represent radicals from the series comprising phenyl, phenyloxy and phenylthio which are optionally substituted by halogen, nitro, cyano, C₁-C₄-alkoxy, C₁-C₄-alkylthio, C₁-C₄-halogenoalkoxy, C₁-C₄-halogenoalkylthio, C₁-C₄-alkyl and/or C₁-C₄-halogenoalkyl;
R⁸ and R⁹ independently of one another represent hydrogen, radicals from the series comprising C₁-C₂₀-alkyl, C₁-C₂₀-alkoxy, C₃-C₁₀-cycloalkyl, C₂-C₈-alkenyl and C₁-C₈-alkoxy-C₁-C₈-alkyl which are optionally substituted by halogen, or represent radicals from the series comprising phenyl and benzyl which are optionally substituted by halogen, C₁-C₂₀-alkyl, C₁-C₂₀-halogenoalkyl and/or C₁-C₂₀-alkoxy, or R⁸ and R⁹ together form a C₂-C₆-alkylene group which can be interrupted by oxygen and/or sulphur;
L represents oxygen or sulphur and
M represents oxygen or sulphur,
with the exception of the compound of the formula

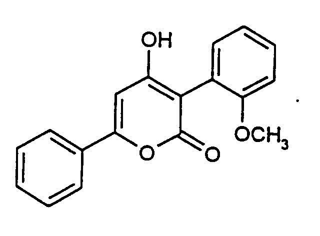

3. Aryl-pyrone derivatives according to Claim 1, in which
A represents hydrogen; halogen; radicals from the series comprising C₁-C₈-alkyl, C₃-C₈-alkenyl, C₃-C₈-alkinyl and C₃-C₈-cycloalkyl, each of which is optionally substituted by halogen; or represents radicals from the series comprising phenyl, naphthyl, hetaryl, phenyl-C₁-C₄-alkyl and hetaryl-C₁-C₄-alkyl, each of which is optionally substituted by halogen, nitro, C₁-C₄-alkyl, C₁-C₄-alkoxy, C₁-C₄-alkylthio, C₁-C₄-halogenoalkyl, C₁-C₄-halogenoalkoxy and/or CN, or represents the groups -COR¹, -CO₂R¹, -CN, -CONR¹R², -SO₂R¹ and -P(O)(OR¹)(OR²), in which
R¹ and R² independently of one another represent hydrogen, radicals from the series comprising C₁-C₈-alkyl and C₃-C₈-alkenyl, which are optionally substituted by halogen; radicals from the series comprising phenyl, hetaryl, phenyl-C₁-C₄-alkyl and hetaryl-C₁-C₄-alkyl, which are optionally substituted by halogen, nitro, C₁-C₄-alkyl, C₁-C₄-alkoxy, C₁-C₄-alkylthio, C₁-C₄-halogenoalkyl, C₁-C₄-halogenoalkoxy and/or CN; or R¹ and R² together represent a C₂-C₆-alkylene group which is optionally interrupted by nitrogen, oxygen or sulphur;
B represents hydrogen; radicals from the series comprising C₁-C₈-alkyl, C₃-C₈-alkenyl, C₃-C₈-alkinyl and C₃-C₈-cycloalkyl which are optionally substituted by halogen, C₁₋₄-alkoxy, C₁₋₄-alkylthio, C₁-C₄-alkylcarbonyloxy or phenyl, or represents radicals from the series comprising phenyl, naphthyl, hetaryl, phenyl-C₁-C₄-alkyl and hetaryl-C₁-C₄-alkyl which are optionally substituted by halogen, CN, nitro, C₁-C₆-alkyl, C₁-C₆-alkoxy, C₁-C₆-alkylthio, C₁-C₆-halogenoalkyl and/or C₁-C₆-halogenoalkoxy; and
A and B together represent a C₂-C₆-alkylene or C₂-C₆-alkenylene group which is optionally substituted by 1 to 5 halogeno-C₁-C₄-alkyl, C₁₋₄-alkoxy, C₁-C₄-alkyl and/or halogen and which can be interrupted by, or contain, nitrogen, sulphur, oxygen or -O-CO-,
X represents halogen, C₁-C₄-alkyl or C₁-C₄-alkoxy;
Y represents hydrogen, halogen, C₁-C₄-alkyl, C₁-C₄-alkoxy or C₁-C₄-halogenoalkyl;
Z represents hydrogen, halogen, C₁-C₄-alkyl or C₁-C₄-alkoxy;
n represents 1 or 2;
G represents hydrogen, -COR³, 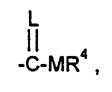 -SO₂-R⁵, 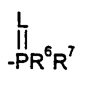 and -CONR⁸R⁹,
in which
R³ represents radicals from the series comprising C₁-C₁₆-alkyl, C₂-C₁₆-alkenyl, C₁-C₆-alkoxy-C₁-C₆-alkyl, C₁-C₆-alkylthio-C₁-C₆-alkyl, C₁-C₆-polyalkoxy-C₁-C₆-alkyl and C₃-C₇-cycloalkyl which is optionally interrupted by oxygen and/or sulphur, each of these radicals optionally being substituted by halogen; phenyl which is optionally substituted by halogen, nitro, C₁-C₄-alkyl, C₁-C₄-alkoxy, C₁-C₃-halogenoalkyl and/or C₁-C₃-halogenoalkoxy; phenyl-C₁-C₄-alkyl which is optionally substituted by halogen, C₁-C₄-alkyl, C₁-C₄-alkoxy, C₁-C₃-halogenoalkyl and/or C₁-C₃-halogenoalkoxy; hetaryl which is optionally substituted by halogen and/or C₁-C₆-alkyl; phenoxy-C₁-C₅-alkyl which is optionally substituted by halogen and/or C₁-C₄-alkyl, or hetaryloxy-C₁-C₅-alkyl which is optionally substituted by halogen, amino and/or C₁-C₄-alkyl;
R⁴ represents radicals from the series comprising C₁-C₁₆-alkyl, C₂-C₁₆-alkenyl, C₁-C₁₆-alkoxy-C₁-C₆-alkyl and C₁-C₆-polyalkoxy-C₁-C₆-alkyl which are optionally substituted by halogen or represents radicals from the series comprising phenyl and benzyl which are optionally substituted by halogen, nitro, C₁-C₄-alkyl, C₁-C₃-alkoxy and/or C₁-C₃-halogenoalkyl;
R⁸ and R⁹ independently of one another represent hydrogen, radicals from the series comprising C₁-C₁₆-alkyl, C₁-C₁₆-alkoxy, C₃-C₁₆-cycloalkyl, C₂-C₆-alkenyl and C₁-C₆-alkoxy-C₁-C₆-alkyl which are optionally substituted by halogen, or represent radicals from the series comprising phenyl and benzyl which are optionally substituted by halogen, C₁-C₆-alkyl, C₁-C₆-halogenoalkyl and/or C₁-C₆-alkoxy, or R⁸ and R⁹ together form a C₂-C₆-alkylene group which can be interrupted by oxygen and/or sulphur;
L represents oxygen or sulphur; and
M represents oxygen or sulphur,
with the exception of the compound of the formula

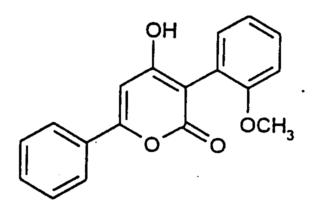

4. 3-Aryl-pyrone derivatives according to Claim 1, in which
A represents hydrogen, radicals from the series comprising C₁-C₄-alkyl, phenyl and benzyl which are optionally substituted by halogen, or A and B together form a C₃-C₅-alkenyl group which is optionally interrupted by oxygen or sulphur;
B represents hydrogen, C₁-C₄-alkyl which is optionally substituted by halogen, CN, methoxy or methylthio or represents radicals from the series comprising phenyl, furyl, thiazolyl and pyridyl which are optionally substituted by halogen, nitro, C₁-C₄-alkyl and/or C₁-C₄-alkoxy; and A and B together form the groups -(CH₂)₃-, -(CH₂)₄-, -(CH₂)₅-, -CH₂-CH(CH₃)(CH₂)₂-, -CH₂O-(CH₂)₂ or -CH₂S(CH₂)₂-, each of which can be substituted by CF₃, Cl or methoxy;
X represents methyl;
Y represents methyl;
Z represents methyl; and
G represents hydrogen or COR³ in which
R³ represents C₁-C₆-alkyl or C₁-C₆-alkoxy.

5. Process for the preparation of the 3-aryl-pyrone derivatives according to Claim 1, characterised in that
a) to prepare the compounds in which G represents hydrogen,
carbonyl compounds of the general formula (II) 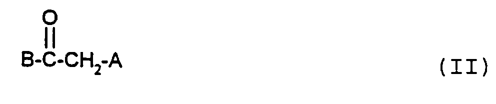 in which
A and B have the abovementioned meaning, are reacted with acid halides derived from ketene of the general formula (III) 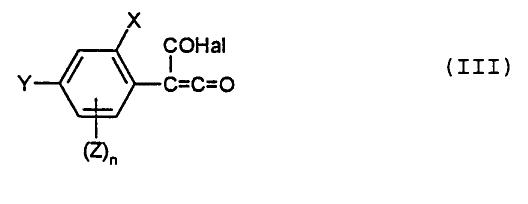 in which
X, Y, Z and n have the abovementioned meanings and
Hal represents halogen
in the presence of a diluent and, if appropriate, in the presence of an acid acceptor; and
b) to prepare compounds of the general formula (I) in which G represents -COR³,
compounds of the general formula (I) in which G represents hydrogen (obtainable by variant a)) are reacted
α) with acid halides of the general formula (IV) 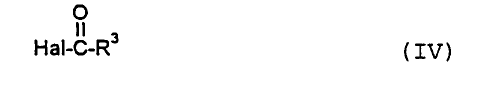 in which
R³ has the abovementioned meaning and
Hal represents halogen, or
β) with carboxylic anhydrides of the general formula (V) 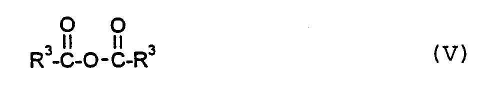 in which
R³ has the abovementioned meaning,
if appropriate in the presence of a diluent and if appropriate in the presence of an acid-binding agent; and
c) to prepare compounds of the general formula (I) in which G represents -C(L)-MR⁴ where L denotes oxygen,
compounds of the general formula (I) in which G represents hydrogen (obtainable by variant a))
are reacted with compounds of the general formula (VI) 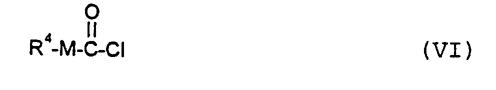 in which
R⁴ and M have the abovementioned meanings,
if appropriate in the presence of a diluent and if appropriate in the presence of an acid-binding agent; and
d) to prepare compounds of the general formula (I) in which G represents -C(L)-MR⁴, where L denotes sulphur,
compounds of the general formula (I) in which G represents hydrogen (obtainable by variant a)) are reacted
α) with compounds of the general formula (VII) 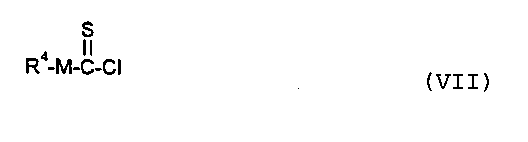 in which
R⁴ and M have the abovementioned meanings,
if appropriate in the presence of a diluent and, if appropriate, in the presence of an acid-binding agent, or
β) with carbon disulphide (CS₂) and subsequently with alkyl halides of the general formula (VIII)
R⁴-Hal¹ (VIII)
in which
R⁴ has the abovementioned meaning and
Hal¹ represents halogen,
if appropriate in the presence of a diluent and, if appropriate, in the presence of an acid-binding agent; and
e) to prepare compounds of the general formula (I) in which G represents -SO₂R⁵,
compounds of the general formula (I) in which G represents hydrogen (obtainable by variant a))
are reacted with sulphonyl chlorides of the general formula (IX)
R⁵-SO₂-Cl (IX)
in which
R⁵ has the abovementioned meaning,
if appropriate in the presence of a diluent and, if appropriate, in the presence of an acid-binding agent; and
f) to prepare compounds of the general formula (I) in which G represents -P(L)R⁶R⁷,
compounds of the general formula (I) in which G represents hydrogen (obtainable by variant a))
are reacted with compounds of the general formula (X) 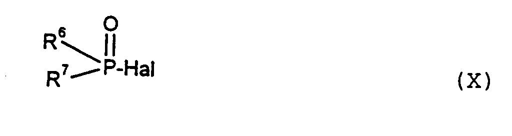 in which
R⁶, R⁷ and L have the abovementioned meanings and
Hal represents halogen,
if appropriate in the presence of a diluent and, if appropriate, in the presence of an acid-binding agent; and
g) to prepare compounds of the general formula (I) in which G represents a metal ion equivalent or an ammonium ion,
compounds of the general formula (I) in which G represents hydrogen (obtainable by variant a))
are reacted with metal hydroxides or amines; and
h) to prepare compounds of the general formula (I) in which G represents -C(L)NR⁸R⁹,
compounds of the general formula (I) in which G represents hydrogen (obtainable by variant a)) are reacted
α) with compounds of the general formula (XI)
R⁸-N=C=L (XI)
in which
R⁸ and L have the abovementioned meanings,
if appropriate in the presence of a diluent and, if appropriate, in the presence of a catalyst; or
β) with compounds of the general formula (XII) 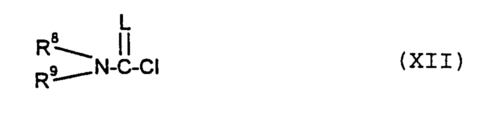 in which
R⁸, R⁹ and L have the abovementioned meanings,
if appropriate in the presence of a diluent and, if appropriate, in the presence of an acid-binding agent.

6. Pesticides, characterized in that they contain at least one compound of the formula (I) according to Claim 1.

7. Arthropodicidal, nematicidal and herbicidal agents, characterised in that they contain at least one compound of the formula (I).

8. Use of compounds of the formula (I) according to Claim 1 for combating pests in plant protection, in the domestic field, in the hygiene field and in the protection of stored products.

9. Use of compounds of the formula (I) according to Claim 1 for combating arthropods, nematodes and undesired plant growth in plant protection, in the domestic field, in the hygiene field and in the protection of stored products.

10. Method of combating pests in plant protection, in the domestic field, in the hygiene field and in the protection of stored products, characterized in that compounds of the formula (I) according to Claim 1 are allowed to act on the pests and/or their environment.

11. Process for the preparation of pesticides, characterized in that compounds of the formula (I) according to Claim 1 are mixed with extenders and/or surface-active agents.

12. Use of compounds of the formula (I) according to Claim 1 for the preparation of pesticides for combating ecto- and endoparasites.

## Revendications

1. Dérivés de 3-aryl-pyrones de formule générale (I) 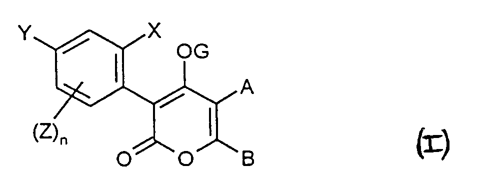 dans laquelle
A représente de l'hydrogène, un halogène, des restes éventuellement substitués de la série alkyle, cycloalkyle, alcényle, alcynyle, arylalkyle, aryle, hétarylalkyle et hétaryle ou les groupes -COR¹, -CO₂R¹, -CN, -CONR¹R², -SO₂R¹ et -P(O)(OR¹)(OR²), où
R¹ et R² représentent, indépendamment l'un de l'autre, de l'hydrogène ou des restes eventuellement substitués de la série alkyle, alcényle, arylalkyle, aryle, hétarylalkyle et hétaryle, ou bien
R¹ et R² forment ensemble un groupe alkylène éventuellement substitué, qui peut être interrompu par un ou plusieurs hétéroatomes ;
B représente de l'hydrogène ou des restes éventuellement substitués de la série alkyle, cycloalkyle, alcényle, alcynyle, arylalkyle, aryle, hétarylalkyle et hétaryle ;
A et B forment ensemble un groupe alkylène ou alcénylène éventuellement substitué, qui peut être interrompu par un ou plusieurs hétéroatomes ou hétérogroupes ou qui peut en contenir ;
X représente un halogène, un groupe alkyle ou alkoxy ;
Y est de l'hydrogène, un halogène, un groupe alkyle, halogénalkyle ou alkoxy ;
Z représente de l'hydrogène, un halogène, un groupe alkyle ou alkoxy,
n représente un nombre entier égal à 1, 2 ou 3 ; et
G représente de l'hydrogène, un équivalent d'ion de métal, un ion ammonium ou l'un des groupes -COR³, 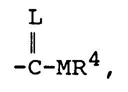 -SO₂-R⁵, 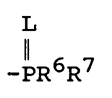 et -CONR⁸R⁹,
dans lesquels
R³ représente des restes éventuellement substitués de la série alkyle, alcényle, alkoxyalkyle, polyalkoxyalkyle, alkylthioalkyle, cycloalkyle éventuellement interrompu par un ou plusieurs hétéroatomes, arylalkyle, aryle, hétarylalkyle, hétaryle, aryloxyalkyle et hétaryloxyalkyle ;
R⁴ représente des restes éventuellement substitués de la série alkyle, alcényle, alkoxyalkyle, polyalkoxyalkyle, aryle et arylalkyle ;
R⁵, R⁶ et R⁷ représentent, indépendamment les uns des autres, des restes éventuellement substitués de la série alkyle, alkoxy, alkylthio, alkylamino, dialkylamino, alcénylthio, alcynylthio, cycloalkylthio, aryle, aryloxy et arylthio ;
R⁸ et R⁹ représentent, indépendamment l'un de l'autre, de l'hydrogène ou des restes éventuellement substitués de la série alkyle, alcényle, cycloalkyle, alkoxyalkyle, aryle ou arylalkyle, ou forment ensemble un groupe alkylène éventuellement substitué, qui peut être interrompu par un ou plusieurs hétéroatomes ou hétérogroupes ;
L est de l'oxygène ou du soufre ; et
M est de l'oxygène ou du soufre,
à l'exception du composé de formule

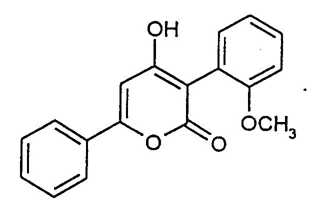

2. Dérivés d'aryl-pyrones suivant la revendication 1, dans lesquels
A représente de l'hydrogène, un halogène, des restes éventuellement substitués par un halogène de la série alkyle en C₁ à C₁₀, alcényle en C₃ à C₁₀, alcynyle en C₃ à C₁₀ et cycloalkyle en C₃ à C₁₀, des restes éventuellement substitués par un halogène, un radical nitro, alkyle en C₁ à C₈, alkoxy en C₁ à C₈, alkylthio en C₁ à C₈, halogénalkyle en C₁ à C₈, halogénalkoxy en C₁ à C₈ et/ou CN de la série phényle, naphtyle, hétaryle, phényl-(alkyle en C₁ à C₆) et hétaryl-(alkyle en C₁ à C₆) ou les groupes -COR¹, -CO₂R¹, -CN, -CONR¹R², -SO₂R¹ et -P(O)(OR¹)(OR²), où
R¹ et R² représentent, indépendamment, l'un de l'autre, de l'hydrogène, des restes, éventuellement substitués par un halogène, de la série alkyle en C₁ à C₁₀ et alcényle en C₃ à C₁₀ ; des restes, éventuellement substitués par un halogène, un radical nitro, alkyle en C₁ à C₈, alkoxy en C₁ à C₈, alkylthio en C₁ à C₈, halogénalkyle en C₁ à C₈, halogénalkoxy en C₁ à C₈ et/ou CN, de la serie phényle, naphtyle, hétaryle, phényl-(alkyle en C₁ à C₆) et hétaryl-(alkyle en C₁ à C₆) ; ou bien R¹ et R² forment ensemble un groupe alkylène en C₂ à C₇ éventuellement interrompu par de l'azote, de l'oxygène ou du soufre ;
B reprêsente de l'hydrogène, des restes, éventuellement substitués par un halogène, un radical CN, alkoxy en C₁ à C₆, alkylthio en C₁ à C₆, (alkyle en C₁ à C₆)carbonyloxy ou phényle, de la série alkyle en C₁ à C₁₀, alcényle en C₃ à C₁₀, alcynyle en C₃ à C₁₀ et cycloalkyle en C₃ à C₁₀ et des restes, éventuellement substituté par un halogène, un radical CN, nitro, alkyle en C₁ à C₈, alkoxy en C₁ à C₈, alkylthio en C₁ à C₈, halogénalkyle en C₁ à C₈ et/ou halogénalkoxy en C₁ à C₈, de la série phényle, naphtyle, hétaryle, phényl-(alkyle en C₁ à C₆) et hétaryl-(alkyle en C₁ à C₆), et
A et B forment ensemble un groupe alkylène en C₂ à C₇ ou alcénylène en C₂ à C₇ éventuellement substitué par un radical alkyle en C₁ à C₈, halogénalkyle en C₁ à C₆, alkoxy en C₁ à C₆, alkylthio en C₁ à C₆ et/ou un halogène, qui peut être interrompu par de l'azote, du soufre, de l'oxygène ou un groupe -O-CO- ou qui peut les contenir ;
X représente un halogène, un groupe alkyle en C₁ à C₆ ou alkoxy en C₁ à C₆ ;
Y est de l'hydrogène, un halogène, un groupe alkyle en C₁ à C₆, alkoxy en C₁ à C₆ ou halogénalkyle en C₁ à C₆ ;
Z représente de l'hydrogène, un halogène, un groupe alkyle en C₁ à C₆ ou alkoxy en C₁ à C₆ ;
n a la valeur 1 ou 2 ;
G représente de l'hydrogène, des groupes groupes -COR³, 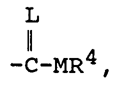 -SO₂-R⁵, 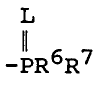 et -CONR⁸R⁹,
où
R³ représente des restes, éventuellement substitués par un halogène, de la série alkyle en C₁ à C₂₀, alcényle en C₂ à C₂₀ (alkoxy en C₁ à C₈)-(alkyle en C₁ à C₈), (alkylthio en C₁ à C₈)-(alkyle en C₁ à C₈), (polyalkoxy en C₁ à C₈)-(alkyle en C₁ à C₈) et un groupe cycloalkyle en C₃ à C₈ éventuellement interrompu par de l'oxygène et/ou du soufre ; un reste phényle éventuellement substitué par un halogène, un radical nitro, alkyle en C₁ à C₆, alkoxy en C₁ à C₆, halogénalkyle en C₁ à C₆ et/ou halogénalkoxy en C₁ à C₆ ; un reste phényl-(alkyle en C₁ à C₆) éventuellement substitué par un halogène, un radical alkyle en C₁ à C₆, alkoxy en C₁ à C₆, halogénalkyle en C₁ à C₆ et/ou halogénalkoxy en C₁ à C₆ ; un reste hétaryle éventuellement substitué par un halogène et/ou un radical alkyle en C₁ à C₆ ; un reste phénoxy-(alkyle en C₁ à C₆) éventuellement substitué par un halogène et/ou un radical alkyle en C₁ à C₆ ou un reste hétaryloxy-(alkyle en C₁ à C₆) éventuellement substitué par un halogène, un radical amino et/ou alkyle en C₁ à C₆ ;
R⁴ représente des restes, éventuellement substitués par un halogène, de la série alkyle en C₁ à C₂₀, alcényle en C₂ à C₂₀ (alkoxy en C₁ à C₈)-(alkyle en C₁ à C₈), (polyalkoxy en C₁ à C₈)-(alkyle en C₁ à C₈) ou des restes, éventuellement substitués par un halogène, un radical nitro, alkyle en C₁ à C₆, alkoxy en C₁ à C₆, et/ou halogénalkyle en C₁ à C₆, de la série phényle et benzyle ;
R⁵, R⁶ et R⁷ représentent, indépendamment les uns des autres, des restes éventuellement substitués par un halogène, de la série alkyle en C₁ à C₈, alkoxy en C₁ à C₈, alkylthio en C₁ à C₈, alkylamino en C₁ à C₈, di-(alkyle en C₁ à C₈)amino, alcénylthio en C₂ à C₅, alcynylthio en C₂ à C₅, cycloalkylthio en C₃ à C₇ ou des restes, éventuellement substitués par un halogène, un radical nitro, cyano, alkoxy en C₁ à C₄, alkylthio en C₁ à C₄, halogénalkoxy en C₁ à C₄, halogénalkylthio en C₁ à C₄, alkyle en C₁ à C₄ et/ou halogénalkyle en C₁ à C₄, de la série phényle, phénoxy et phénylthio ;
R⁸ et R⁹ représentent, indépendamment l'un de l'autre, de l'hydrogène, des restes, éventuellement substitués par un halogène, de la série alkyle en C₁ à C₂₀, alkoxy en C₁ à C₂₀, cycloalkyle en C₃ à C₁₀, alcényle en C₂ à C₈, (alkoxy en C₁ à C₈)-(alkyle en C₁ à C₈) ou des restes, éventuellement substitués par un halogène, un radical alkyle en C₁ à C₂₀, halogénalkyle en C₁ à C₂₀ et/ou alkoxy en C₁ à C₂₀, de la série phényle et benzyle ou bien R⁸ et R⁹ forment ensemble un groupe alkylène en C₂ à C₆ qui peut être interrompu par de l'oxygène et/ou du soufre ;
L représente de l'oxygène ou du soufre ; et
M représente de l'oxygène ou du soufre,
à l'exception du composé de formule

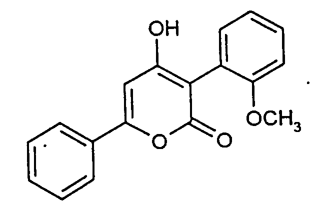

3. Dérivés d'aryl-pyrones suivant la revendication 1, dans lesquels
A représente de l'hydrogène ; un halogène ; des restes, éventuellement substitués par un halogène, de la série alkyle en C₁ à C₈, alcényle en C₃ à C₈, alcynyle en C₃ à C₈ et cycloalkyle en C₃ à C₈ ; des restes, éventuellement substitués par un halogène, un radical nitro, alkyle en C₁ à C₄, alkoxy en C₁ à C₄, alkylthio en C₁ à C₄, halogénalkyle en C₁ à C₄, halogénalkoxy en C₁ à C₄ et/ou CN, de la série phényle, naphtyle, hétaryle, phényl-(alkyle en C₁ à C₄) et hétaryl-(alkyle en C₁ à C₄) ou les groupes -COR¹, -CO₂R¹, -CN, -CONR¹R², -SO₂R¹ et -P(O)(OR¹)(OR²), dans lesquels
R¹ et R² représentent, indépendamment, l'un de l'autre, de l'hydrogène, des restes, éventuellement substitués par un halogène, de la série alkyle en C₁ à C₈ et alcényle en C₃ à C₈ ; des restes, éventuellement substitués par un halogène, un radical nitro, alkyle en C₁ à C₄, alkoxy en C₁ à C₄, alkylthio en C₁ à C₄, halogénalkyle en C₁ à C₄, halogénalkoxy en C₁ à C₄ et/ou CN, de la série phényle, hétaryle, phényl-(alkyle en C₁ à C₄) et hétaryl-(alkyle en C₁ à C₄) ; ou bien R¹ et R² forment ensemble un groupe alkylène en C₂ à C₆ éventuellement interrompu par de l'azote, de l'oxygène ou du soufre ;
B représente de l'hydrogène, des restes, éventuellement substitués par un halogène, un radical alkoxy en C₁ à C₄, alkylthio en C₁ à C₄, (alkyle en C₁ à C₄)carbonyloxy ou phényle, de la série alkyle en C₁ à C₈, alcényle en C₃ à C₈, alcynyle en C₃ à C₈ et cycloalkyle en C₃ à C₈ ou des restes, éventuellement substitués par un halogène, un radical CN, nitro, alkyle en C₁ à C₆, alkoxy en C₁ à C₆, alkylthio en C₁ à C₆, halogénalkyle en C₁ à C₆ et/ou halogénalkoxy en C₁ à C₆, de la série phényle, naphtyle, hétaryle, phényl-(alkyle en C₁ à C₄) et hétaryl-(alkyle en C₁ à C₄) ; et
A et B forment ensemble un groupe alkylène en C₂ à C₆ ou alcénylène en C₂ à C₆ portant éventuellement 1 à 5 substituants halogénalkyle en C₁ à C₄, alkoxy en C₁ à C₄, alkyle en C₁ à C₄ et/ou halogéno, qui peut être interrompu par de l'azote, du soufre, de l'oxygène ou un groupe -O-CO- ou qui peut les contenir,
X représente un halogène, un groupe alkyle en C₁ à C₄ ou alkoxy en C₁ à C₄ ;
Y représente de I'hydrogène, un halogène, un groupe alkyle en C₁ à C₄, alkoxy en C₁ à C₄ ou halogénalkyle en C₁ à C₄ ;
Z représente de l'hydrogène, un halogène, un groupe alkyle en C₁ à C₄ ou alkoxy en C₁ à C₄ ;
n a la valeur 1 ou 2 ;
G représente de l'hydrogène, des groupes groupes -COR³, 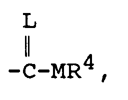 -SO₂-R⁵, 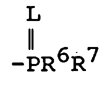 et -CONR⁸R⁹,
où
R³ représente des restes, éventuellement substitués par un halogène, de la série alkyle en C₁ à C₁₆, alcényle en C₂ à C₁₆ (alkoxy en C₁ à C₆)-(alkyle en C₁ à C₆), (alkylthio en C₁ à C₆)-(alkyle en C₁ à C₆), (polyalkoxy en C₁ à C₆)-(alkyle en C₁ à C₆) et cycloalkyle en C₃ à C₇ éventuellement interrompu par de l'oxygène et/ou du soufre ; un reste phényle éventuellement substitué par un halogène, un radical nitro, alkyle en C₁ à C₄, alkoxy en C₁ à C₄, halogénalkyle en C₁ à C₃ et/ou halogénalkoxy en C₁ à C₃ ; un reste phényl-(alkyle en C₁ à C₄) éventuellement substitué par un halogène, un radical alkyle en C₁ à C₄, alkoxy en C₁ à C₄, halogénalkyle en C₁ à C₃ et/ou halogénalkoxy en C₁ à C₃ ; un reste hétaryle éventuellement substitué par un halogène et/ou un radical alkyle en C₁ à C₆ ; un reste phénoxy-(alkyle en C₁ à C₅) éventuellement substitué par un halogène et/ou un radical alkyle en C₁ à C₄ ou un reste hétaryloxy-(alkyle en C₁ à C₅) éventuellement substitué par un halogène, un radical amino et/ou alkyle en C₁ à C₄ ;
R⁴ représente des restes, éventuellement substitués par un halogène, de la série alkyle en C₁ à C₁₆, alcényle en C₂ à C₁₆, (alkoxy en C₁ à C₁₆)-(alkyle en C₁ à C₆), (polyalkoxy en C₁ à C₆)-(alkyle en C₁ à C₆) ou des restes, éventuellement substitués par un halogène, un radical nitro, alkyle en C₁ à C₄, alkoxy en C₁ à C₃ et/ou halogénalkyle en C₁ à C₃, de la série phényle et benzyle ;
R⁸ et R⁹ représentent, indépendamment l'un de l'autre, de l'hydrogène, des restes, éventuellement substitués par un halogène, de la série alkyle en C₁ à C₁₆, alkoxy en C₁ à C₁₆, cycloalkyle en C₃ à C₁₆, alcényle en C₂ à C₆, (alkoxy en C₁ à C₆)-(alkyle en C₁ à C₆) ou des restes, éventuellement substitués par un halogène, un radical alkyle en C₁ à C₆, halogénalkyle en C₁ à C₆ et/ou alkoxy en C₁ à C₆, de la série phényle et benzyle, ou bien R⁸ et R⁹ forment ensemble un groupe alkylène en C₂ à C₆ qui peut être interrompu par de l'oxygène et/ou du soufre ;
L est de l'oxygène ou du soufre ; et
M est de l'oxygène ou du soufre,
à l'exception du composé de formule

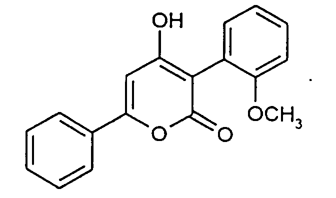

4. Dérivés de 3-aryl-pyrones suivant la revendication 1, dans lesquels
A représente de l'hydrogène, un halogène, des restes, éventuellement substitués par un halogène, de la série alkyle en C₁ à C₄, phényle et benzyle ou bien A et B forment ensemble un groupe alcényle en C₃ à C₅ éventuellement interrompu par de l'oxygène ou du soufre ;
B représente de l'hydrogène, un groupe alkyle en C₁ à C₄ éventuellement substitué par un halogène, un radical CN, méthoxy, méthylthio ou des restes, éventuellement substitués par un halogène, un radical nitro, alkyle en C₁ à C₄ et/ou alkoxy en C₁ à C₄, de la série phényle, furyle, thiazolyle et pyridyle : et A et B forment ensemble les groupes -(CH₂)₃-, -(CH₂)₄-, -(CH₂)₅-, -CH₂-CH(CH₃)(CH₂)₂-, -CH₂O-(CH₂)₂- ou -CH₂S(CH₂)₂-, qui peuvent être substitués par un radical CF₃, Cl ou méthoxy ;
X est un groupe méthyle ;
Y est un groupe méthyle ;
Z est un groupe méthyle ; et
G représente de l'hydrogène ou un groupe COR³, dans lequel
R³ est un groupe alkyle en C₁ à C₆ ou alkoxy en C₁ à C₆.

5. Procédé de production des dérivés de 3-aryl-pyrones suivant la revendication 1, caractérisé en ce que :
a) pour l'obtention des composés dans lesquels G est de l'hydrogène,
on fait réagir des composés carbonyliques de formule générale (II) 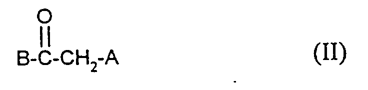 dans laquelle
A et B ont la définition indiquée ci-dessus, avec des halogénures d'acides cétène-carboxyliques de formule générale (III) 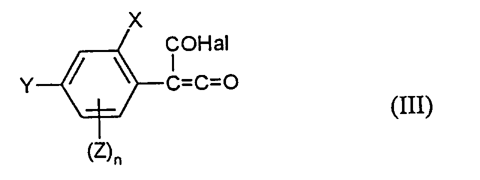 dans laquelle
X, Y, Z et n ont les définitions indiquées ci-dessus et
Hal représente un halogène, en présence d'un diluant et, le cas échéant, en présence d'un accepteur d'acide ; et
b) pour l'obtention des composés de formule générale (I) dans laquelle G est un groupe -COR³,
on fait réagir des composés de formule générale (I), dans lesquels G représente de l'hydrogène (pouvant être obtenus selon la variante a))
α) avec des halogénures d'acides de formule générale (IV) 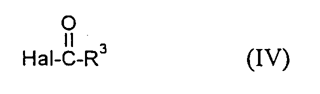 dans laquelle
R³ a la définition indiquée ci-dessus et
Hal représente un halogène, ou bien
β) avec des anhydrides d'acides carboxyliques de formule générale (V) 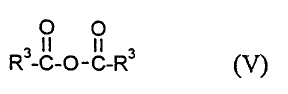 dans laquelle
R³ a la définition indiquée ci-dessus, le cas échéant en présence d'un diluant et en la présence éventuelle d'un accepteur d'acide ; et
c) pour l'obtention des composés de formule générale (I), dans laquelle G est un groupe -C(L)-MR⁴, où L est de l'oxygène,
on fait réagir des composés de formule générale (I) dans lesquels G représente de l'hydrogène (pouvant être obtenus d'après la variante a)),
avec des composés de formule générale (VI) 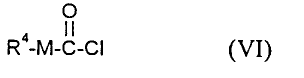 dans laquelle
R⁴ et M ont les définitions indiquées ci-dessus, le cas échéant, en présence d'un diluant et en la présence éventuelle d'un accepteur d'acide ; et
d) pour l'obtention des composés de formule générale (I) dans lesquels G est un groupe -C(L)-MR⁴, où L représente du soufre,
on fait réagir des composés de formule générale (I) dans lesquels G représente de l'hydrogène (pouvant être obtenus d'après la variante a)),
α) avec des composés de formule générale (VII) 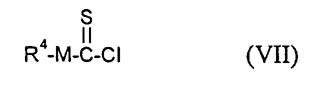 dans laquelle
R⁴ et M ont les définitions indiquées ci-dessus, le cas échéant en présence d'un diluant et en la présence éventuelle d'un accepteur d'acide, ou bien
β) avec du sulfure de carbone, puis avec des halogénures d'alkyle de formule générale (VIII)
R⁴-Hal¹ (VIII)
dans laquelle
R⁴ a la définition indiquée ci-dessus et
Hal¹ est un halogène,
le cas échéant en présence d'un diluant et en la présence éventuelle d'un accepteur d'acide ; et
e) pour l'obtention de composés de formule générale (I), dans laquelle G est un groupe -SO₂R⁵,
on fait réagir des composés de formule générale (I), dans lesquels G représente de l'hydrogène (pouvant être obtenus d'après la variante a)),
avec des chlorures d'acide sulfonique de formule générale (IX)
R⁵-SO₂-Cl (IX)
dans laquelle
R⁵ a la définition indiquée ci-dessus, le cas échéant en présence d'un diluant et en la présence éventuelle d'un accepteur d'acide ; et
f) pour l'obtention de composés de formule générale (I), dans lesquels G est un groupe -P(L)R⁶R⁷,
on fait réagir des composés de formule générale (I) dans lesquels G représente de l'hydrogène (pouvant être obtenus d'après la variante a)),
avec des composés de formule générale (X) 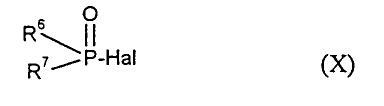 dans laquelle
R⁶, R⁷ et L ont les définitions indiquées ci-dessus et
Hal est un halogène,
le cas échéant en présence d'un diluant et la présence éventuelle d'un accepteur d'acide ; et
g) pour l'obtention de composés de formule générale (I), dans lesquels G est un équivalent d'ions de métal ou un ion ammonium,
on fait réagir des composés de formule générale (I), dans lesquels G représente de l'hydrogène (pouvant être obtenus d'après la variante a)),
avec des hydroxydes de métaux ou des amines ; et
h) pour l'obtention de composés de formule générale (I), dans lesquels G est un groupe -C(L)NR⁸R⁹,
on fait réagir des composés de formule générale (I), dans lesquels G est de l'hydrogène (pouvant être obtenus d'après la variante a)),
α) avec des composés de formule générale (XI)
R⁸-N=C=L (XI) (XI)
dans laquelle
R⁸ et L ont les définitions indiquées ci-dessus, le cas échéant en présence d'un diluant et en la présence éventuelle d'un catalyseur ; ou bien
β) avec des composés de formule générale (XII) 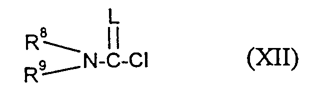 dans laquelle
R⁸, R⁹ et L ont les définitions indiquées ci-dessus,
le cas échéant en présence d'un diluant et en la présence éventuelle d'un accepteur d'acide.

6. Compositions pesticides, caractérisées par une teneur en au moins un composé de formule (I) suivant la revendication 1.

7. Compositions arthropodicides, nématicides et herbicides, caractérisées par une teneur en au moins un composé de formule (I).

8. Utilisation de composés de formule (I) suivant la revendication 1 pour combattre des parasites dans la protection des plantes, dans le domaine de l'entretien, dans le secteur de l'hygiène et dans la protection des denrées entreposées.

9. Utilisation de composés de formule (I) suivant la revendication 1 pour combattre des parasites dans la protection des plantes, dans le domaine de l'entretien, dans le secteur de l'hygiène et dans la protection des denrées entreposées.

10. Procédé pour combattre des parasites dans la protection des plantes, dans le domaine de l'entretien, dans le secteur de l'hygiène et dans la protection des denrées entreposées, caractérisé en ce qu'on fait agir des composés de formule (I) suivant la revendication 1 sur les parasites et/ou sur leur milieu.

11. Procédé de préparation de compositions pesticides, caractérisé en ce qu'on mélange des composés de formule (I) suivant la revendication 1 avec des diluants et/ou des agents tensio-actifs.

12. Utilisation de composés de formule (I) suivant la revendication 1 pour la préparation de compositions pesticides destinées à combattre des ectoparasites et des endoparasites.
